(19) Europäisches Patentamt
European Patent Office
Office européen des brevets

(11) Numéro de publication : **0 004 493**
**B1**

(12) **FASCICULE DE BREVET EUROPÉEN**

(45) Date de publication du fascicule du brevet : **06.05.81**

(51) Int. Cl.³ : **C 07 D315/00**, C 07 D307/93, C 07 B 19/00

(21) Numéro de dépôt : **79400144.6**

(22) Date de dépôt : **06.03.79**

(54) **Nouveaux éthers dont les restes organiques comportent des atomes chiraux, leur procédé de préparation et leur application au dédoublement des alcools, des phénols ou de certains composés de structure lactonique.**

(30) Priorité : **17.03.78 FR 7807778**

(43) Date de publication de la demande : **03.10.79 (Bulletin 79/20)**

(45) Mention de la délivrance du brevet : **06.05.81 Bulletin 81/18**

(84) Etats contractants désignés : **BE CH DE FR GB IT NL SE**

(56) Documents cités : **FR - A - 1 580 474**

(73) Titulaire : **ROUSSEL-UCLAF**
**102, route de Noisy Boîte postale no.9**
**F-93230-Romainville (FR)**

(72) Inventeur : **Martel, Jacques**
**15 rue Douvillez**
**F-93140 Bondy (FR)**
Inventeur : **Tessier, Jean**
**30 rue Jean Moulin**
**F-94300 Vincennes (FR)**
Inventeur : **Teche, André**
**51 avenue Joliot Curie**
**F-92000 Nanterre (FR)**

(74) Mandataire : **Burlot, Pierre et al**
**Boite postale no 9 102, route de Noisy**
**F-93230 Romainville (FR)**

**EP 0 004 493 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

Nouveaux éthers dont les restes organiques comportent des atomes chiraux, leur procédé de préparation et leur application au dédoublement des alcools, des phénols ou de certains composés de structure lactonique

La présente invention concerne de nouveaux éthers dont les restes organiques comportent des atomes chiraux, leur procédé de préparation et leur application au dédoublement des alcools, des phénols ou de certains composés de structure lactonique.

L'invention a pour objet les composés de formule générale I :

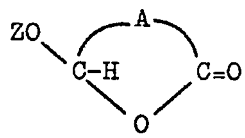

(I)

formule dans laquelle le symbole A représente une chaîne hydrocarbonée comportant de 1 à 10 chaînons, cette chaîne pouvant comporter un ou plusieurs hétéroatomes, une ou plusieurs insaturations, l'ensemble des chaînons constitutifs de la chaîne pouvant représenter un système mono ou polycyclique, y compris un système du type spiro ou endo, la chaîne A pouvant comporter un ou plusieurs atomes chiraux ou bien la copule lactonique pouvant présenter une chiralité due à la configuration spatiale dissymétrique de l'ensemble de la molécule, et le symbole Z représente soit un reste d'alcool primaire, secondaire ou tertiaire, comportant au moins un carbone asymétrique soit un reste phénolique substitué comportant au moins un carbone asymétrique, soit un reste alcoolique ou phénolique substitué, dont la chiralité est due à la configuration spatiale dissymétrique de l'ensemble de la molécule, étant entendu que Z ne peut représenter un radical (R) ou (S) α-cyano 3-phénoxy benzyle lorsque A représente une chaîne hydrocarbonée de structure :

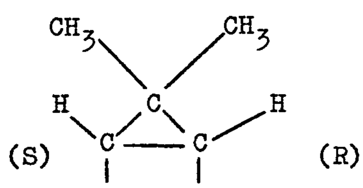

Parmi les composés de formule (I) de l'invention, on peut citer ceux dans lesquels la chaîne A comporte un ou plusieurs carbones asymétriques et dont la structure chimique impose à l'hydroxyle une disposition spatiale univoque. Dans ce type de composés, les deux atomes ou radicaux différents qui substituent des atomes de carbone de la chaîne sont choisis dans l'un ou l'autre des groupes suivants :

a) le groupe constitué par l'hydrogène, les atomes d'halogène, le groupement nitro, les radicaux alcoyles comportant de 1 à 10 atomes de carbone, les radicaux cycloalcoyles comportant de 3 à 6 atomes de carbone, le radical phényle, les radicaux phényles substitués par au moins un des éléments du groupe constitué par les atomes d'halogènes, les radicaux alcoyles comportant de 1 à 6 atomes de carbone, le groupement carboxyle, le groupement nitrile, le groupement -CHO, les groupements acyles, le groupement 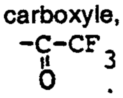 les groupements S-alc et O-alc dans lesquels alc représente un groupement alcoyle comportant de 1 à 6 atomes de carbone,

b) le groupe constitué par les radicaux :

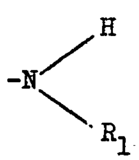

dans lesquels $R_1$ représente de l'hydrogène, un radical alcoyle comportant de 1 à 6 atomes de carbone,

le radical : 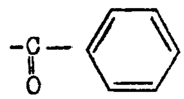 ,

le radical : 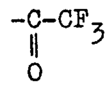 ,

le radical : 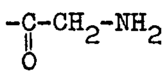 , le radical : 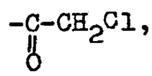

ou un radical 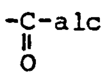 dans lequel alc représente un radical alcoyle comportant de 1 à 6 atomes de carbone,

c) le groupe constitué par les radicaux :

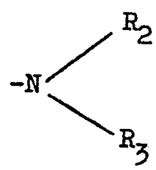

dans lequel $R_2$ et $R_3$, identiques ou différents, représentent un radical alcoyle comportant de 1 à 6 atomes de carbone, ou bien dans lequel $R_2$ et $R_3$ représentent ensemble avec l'atome d'azote auquel ils sont liés, un hétérocycle comportant 6 atomes, ou bien $R_2$ représente un groupement carboxyle et $R_3$ représente un radical benzyle.

Le brevet français 1 580 474 décrit et revendique notamment des esters de formule IV

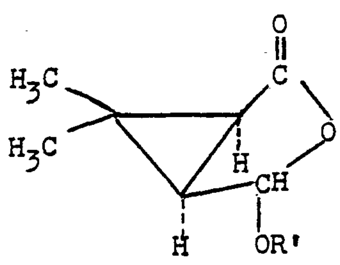

IV

dans laquelle R' représente un radical alcoyle inférieur et notamment le radical méthyle.

La description du brevet cité ne donne aucune indication précise sur la nature de ces radicaux inférieurs et ne comporte en exemple que $R = CH_3$. On doit donc considérer que ne sont envisagés que les composés comportant au plus des radicaux alcoyles inférieurs usuels, tels que méthyle, éthyle ou propyle. Les produits du document cité servent à préparer l'acide cis 3,3-diméthyl 2-formul cyclopropane 1-carboxylique.

La présente invention a pour objet des composés

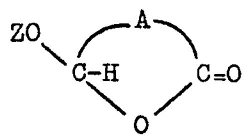

dans lesquels Z comporte au moins un carbone asymétrique. La nature de Z est fondamentale. C'est parce que le groupe Z possède un centre d'asymétrie qu'il est possible d'utiliser les produits de l'invention au dédoublement des alcools chiraux.

Comme famille de composés I particulièrement intéressante on peut signaler :

— celle qui est caractérisée en ce que la chaîne A est une chaîne hydrocarbonée aliphatique comportant 2 ou 3 atomes de carbone ;

— celle qui est caractérisée en ce que la chaîne A est une chaîne hydrocarbonée aliphatique, interrompue par un hétéroatome ;

— celle qui est caractérisée en ce que la chaîne A est une chaîne hydrocarbonée aliphatique comportant une double liaison ;

— celle qui est caractérisée en ce que la chaîne A est une chaîne hydrocarbonée monocyclique comportant de 3 à 6 atomes de carbone, possédant, éventuellement, une insaturation ;

— celle qui est caractérisée en ce que la chaîne A est une chaîne hydrocarbonée bicyclique comportant de 5 à 10 atomes de carbone, possédant, éventuellement, une insaturation.

Comme composé I d'un intérêt particulier on peut citer :

— ceux qui sont caractérisés en ce que la chaîne A a pour structure :

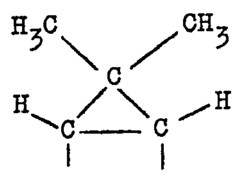

— ceux qui sont caractérisés en ce que la chaîne A a pour structure :

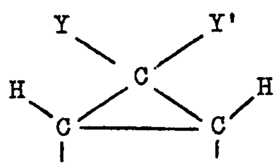

Y et Y', identiques ou différents, représentant des atomes d'hydrogène, de fluor, de chlore ou de brome ou un radical alcoyle comportant de 2 à 6 atomes de carbone, ou bien Y et Y' pouvant former ensemble avec l'atome de carbone auquel ils sont liés un homocycle carboné comportant de 3 à 7 atomes de carbone ;

— ceux qui sont caractérisés en ce que la chaîne A a pour structure :

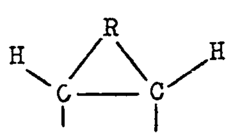

R représentant un atome d'oxygène, de soufre, un groupement -NH ou -NR', R' étant un radical alcoyle comportant de 1 à 6 atomes de carbone ;

— ceux qui sont caractérisés en ce que le symbole Z a pour structure :

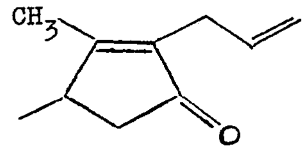

— ceux qui sont caractérisés en ce que le symbole Z a pour structure :

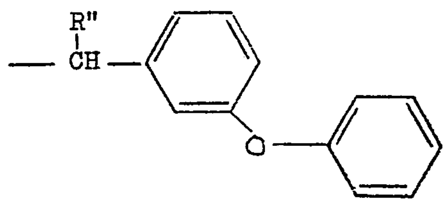

R″ représentant un radical alcoyle comportant de 1 à 6 atomes de carbone, un radical alcényle comportant de 2 à 6 atomes de carbone, un radical alcynyle comportant de 2 à 6 atomes de carbone ou un radical cyano.

Dans les composés de formule I, selon l'invention, le reste Z peut être un reste d'alcool. Dans ce cas, cet alcool peut être primaire, secondaire ou tertiaire, de nature aliphatique, cycloaliphatique ou aromatique, mono ou polycyclique.

Parmi ces alcools on citera, notamment, les cyanhydrines.

Le reste Z peut également être un reste phényle substitué dans lequel le ou les groupements substituants comportent au moins un atome de carbone asymétrique.

Parmi les composés de formule I, on citera plus particulièrement ceux décrits dans les exemples et, notamment, les composés ou mélanges de composés, de formule générale I, dont les noms suivent :

— mélange de (1R, 5S) 6,6 - diméthyl 4(R) - /1 (S) - 2 - méthyl 4 - oxo - 3 - (2 - propen - 1 - yl) - cylcopent - 2 - enyloxy/3 - oxa bicyclo (3.1.0) hexan - 2 - one et de (1R, 5S) 6,6 - diméthyl 4(R) - /1(R) - 2 - méthyl 4 - oxo - 3 - (2 - propen - 1 - yl) cyclopent - 2 - enyloxy/3 - oxa bicyclo (3.1.0) hexan - 2 - one,

— mélange de (1S, 5R) 6,6 - diméthyl 4(R) /1 (S) - 2 - méthyl 4 - oxo 3 - (2 - propen - 1 - yl) cyclopent - 2 - enyloxy/3 - oxa bicyclo (3.1.0) hexan - 2 - one et de (1S, 5R) 6,6 - diméthyl 4(R)/1(R) - 2 - méthyl 4 - oxo 3 - (2 - propen - 1 - yl) cyclopent - 2 - enyloxy/3 - oxa bicyclo (3.1.0) hexan - 2 - one,

— la (1R, 5S) 6,6 - diméthyl 4(R) /1 (S) - 2 méthyl 4 - oxo 3 - (2 - propen - 1 - yl) cyclopent - 2 - enyloxy /3 - oxa bicyclo (3.1.0) hexan - 2 - one,

— la (1S, 5R) 6,6 - diméthyl 4(R) /1(R) - 2 - méthyl 4 - oxo 3 - (2 - propen - 1 - yl) cyclopent - 2 - enyloxy/ 3 - oxa bicyclo (3.1.0) hexan - 2 - one,

— la (1R, 5S) 6,6 - diméthyl 4(R) /3′,3′ - diméthyl butyrolactone 2′ - (S) oxy/3 - oxa bicyclo/3.1.0/ hexan - 2 - one,

— la (1R, 5S) 6,6 - diméthyl 4(R) /3′,3′ - diméthyl butyrolactone 2′ - (R) oxy/3 - oxa bicyclo/3.1.0/hexan - 2 - one, ainsi que le mélange de ces deux derniers composés,

— la (3R) (3aR) (4S) (7R) (7aS) 3 - /(1′R) (2′S) (5′R) 2′ - isopropyl 5′ - méthyl cyclohexanoxy/ - tétrahydro 4,7 - méthano isobenzofuran - 1 - one,

— la (3R) (3aR) (4S) (7R) (7aS) 3 - /(1′S) (2′R) (5′S) 2′ - isopropyl 5′ - méthyl cyclohexanoxy/ - tétrahydro 4,7 - méthano isobenzofuran - 1 - one, ainsi que le mélange de ces deux derniers composés,

— la (1R, 5S) 6,6 - diméthyl 4(R) - /(1′R) (2′S) (5′R) 2′ - isopropyl 5′ - méthyl cyclohexanoxy/3 - oxabicyclo /3.1.0/ hexan - 2 - one,

— la (1R, 5S) 6,6 - diméthyl 4(R) - /(1′S) (2′R) (5′S) 2′ - isopropyl 5′ - méthyl cyclohexanoxy/3 - oxabicyclo /3.1.0/ hexan - 2 - one, ainsi que le mélange de ces deux derniers composés.

L'invention a également pour objet un procédé de préparation des composés de formule générale (I), caractérisé en ce que l'on fait réagir un composé lactonique de formule générale II :

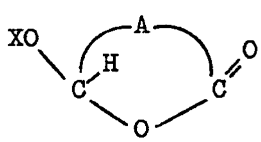

(II)

formule dans laquelle X représente un atome d'hydrogène ou un radical alcoyle comportant de 1 à 4 atomes de carbone et A conserve les significations précitées, en présence d'un acide, avec un alcool ou un phénol substitué de formule générale III :

ZOH (III)

formule dans laquelle Z conserve les significations précitées, pour obtenir :

— ou bien un composé de formule (I), dénommé ($I_A$), dans lequel les atomes chiraux possèdent une configuration bien définie

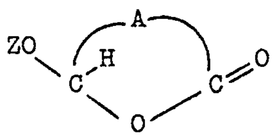

($I_A$)

lorsque la lactone et l'alcool ou le phénol possèdent un ou plusieurs atomes chiraux de configuration bien définie,

— ou bien un mélange de diastéréoisomères, dénommé ($I_B$), lorsque la lactone est un isomère optique bien défini et les centres chiraux de l'alcool ou du phénol ne sont pas tous de configuration univoque,

— ou bien un mélange de diastéréoisomères, dénommé ($I_C$), lorsque l'alcool ou le phénol est un isomère optique bien défini et les atomes chiraux de la lactone ne sont pas tous de configuration univoque, puis sépare par une méthode physique les éthers diastéréoisomères contenus ou bien dans les mélanges du type $I_B$ ou bien dans les mélanges du type $I_C$ et, notamment, l'éther dénommé $I_A$ dont les centres chiraux sont tous de configuration univoque.

L'agent acide en présence duquel on fait réagir l'alcool ou le phénol avec le composé lactonique est, par exemple, choisi dans le groupe constitué par les acides sulfoniques, l'acide perchlorique et l'acide 5-sulfosalicylique.

La réaction de l'alcool ou du phénol avec le composé lactonique est effectuée, de préférence, en éliminant l'eau ou l'alcool formé, par décantation azéotropique, au reflux d'un solvant choisi dans le groupe constitué par les solvants chlorés, les hydrocarbures aromatiques ou aliphatiques et les éthers.

Cette réaction peut également être conduite avantageusement en opérant sous pression réduite et sans solvant.

La séparation des éthers diastéréoisomères (I) est effectuée avantageusement par cristallisation ou chromatographie.

Si le ou les atomes chiraux de la copule lactonique (II) sont chacun de configuration stérique déterminée (R) ou (S), lorsque le ou les atomes de carbone asymétrique de l'alcool ou du phénol substitué (III) sont aussi, individuellement, de configuration stérique déterminée (R) ou (S), on obtient directement avec rétention de configuration, des composés I correspondants dénommés ($I_A$).

Lorsque, dans ce procédé, l'alcool ou le phénol substitué (III) possède un ou plusieurs carbones asymétriques non résolus, on obtient un mélange d'éthers (I) diastéréoisomères, dénommés ($I_B$) que l'on peut alors séparer par un traitement physique, notamment par chromatographie ou par cristallisation dans un solvant (schéma I).

Ce dernier cas est particulièrement intéressant.

Après séparation des éthers diastéréoisomères formant ($I_B$), par exemple après séparation de ($I_A$), une simple hydrolyse ou alcoolyse, comme il est indiqué ci-après, permet d'obtenir les alcools ou phénols substitués dédoublés au niveau du ou des atomes de carbone asymétriques racémiques qu'ils comportaient initialement. Lorsque l'alcool ou le phénol substitué possèdent m centres chiraux non résolus, il se forme 2m diastéréoisomères semblables à $I_A$ qui peuvent, éventuellement, être séparés en individualités telles que $I_A$.

On récupère, par ailleurs, le composé lactonique (II) utilisé, dont le ou les atomes chiraux sont de configuration stérique déterminée (R) ou (S) telle qu'elle existait au départ dans les composés (II).

D'une façon analogue, dans le procédé de préparation des composés I, si le ou les atomes de carbone asymétriques des alcools ou phénols substitués (III) sont de configuration stérique déterminée (R) ou (S), lorsque le ou les atomes chiraux de la copule lactonique sont aussi de configuration stérique déterminée (R) ou (S), on obtient directement, avec rétention de configuration, des composés correspondants dénommés ($I_A$) (schéma II).

Lorsque, dans ce procédé, la copule lactonique est de configuration racémique (RS), du fait d'un ou plusieurs centres chiraux non résolus, on obtient un mélange d'éthers (I) diastéréoisomeres dénommés ($I_C$), que l'on peut alors séparer par un traitement physique, notamment par chromatographie ou par cristallisation dans un solvant.

Ce dernier cas est particulièrement intéressant.

Après séparation des éthers diastéréoisomères formés ($I_C$), par exemple après séparation de ($I_A$), une simple hydrolyse ou alcoolyse, comme il est indiqué ci-après, permet d'obtenir le composé lactonique dédoublé au niveau du ou des atomes chiraux de configuration racémique (RS) qu'il comportait initialement. Lorsque la copule lactonique possède n centres chiraux non résolus, il se forme 2n diastéréoisomères semblables à $I_A$ qui peuvent, éventuellement, être séparés en individualités telles que ($I_A$).

On récupère, par ailleurs, l'alcool ou le phénol substitué (III) utilisé, dont le ou les atomes de carbone asymétriques sont de configuration stérique déterminée (R) ou (S), telle qu'elle existait au départ dans le composé ZOH.

Dans tout ce qui précède, la présence d'un ou plusieurs centres chiraux dédoublés ou non dans les composés (II) ou (III) implique les différentes possibilités suivantes :

— ou bien aucun des centres chiraux ne possède de configuration univoque soit (R), soit (S), et la molécule est alors un mélange de racémates groupant des énantiomères ;

— ou bien une partie des centres chiraux possède une configuration univoque soit (R), soit (S), et la molécule est alors un mélange de diastéréoisomères ;

— ou bien tous les centres chiraux ont une configuration univoque soit (R), soit (S), et la molécule est alors considérée comme un isomère optique bien défini.

En plus de l'asymétrie due à la présence de centres chiraux dans les molécules des composés (II) ou (III), celles-ci peuvent présenter une isomérie géométrique due à la présence d'une ou plusieurs doubles liaisons (E) ou (Z), ainsi qu'une chiralité supplémentaire due à la conformation spatiale de l'ensemble de la molécule dont les atomes constituants peuvent, par exemple, se répartir en deux plans orthogonaux.

Le procédé de l'invention s'applique également dans ce cas et l'on peut, là encore, effectuer un dédoublement d'un ou plusieurs atomes de carbone asymétriques racémiques, présents dans une des copules de l'éther (I) grâce à la chiralité de l'autre partie de l'éther (I).

L'invention a, notamment, pour objet un procédé de préparation des composés de formule générale I, tels que définis précedemment, caractérisé en ce que le composé de formule II est la lactone d'un acide cis 2,2-diméthyl 3-(dihydroxyméthyl) cyclo-propane-1-carboxylique racémique ou optiquement actif, le composé de formule III est la 1-hydroxy 2-méthyl 3-(2-propen-1-yl) cyclopent-2-ène 4-one racémique ou optiquement active et en ce que la séparation éventuelle des composés I diastéréoisomères est effectuée par cristallisation dans un solvant organique, un procédé de préparation des composés de formule générale I, tels que définis précédemment, caractérisé en ce que le composé de formule II est la lactone d'un acide cis 2,2-diméthyl 3-(dihydroxyméthyl) cyclopropane-1-carboxylique racémique ou optiquement actif, le composé de formule III est la 2-hydroxy 3,3-diméthyl butyrolactone (ou pantolactone) racémique ou optiquement active et la séparation éventuelle des composés diastéréoisomères est effectuée par chromatographie, un procédé de préparation des composés de formule générale I, tels que définis précédemment, caractérisé en ce que le composé de formule II est la 3-hydroxy tétrahydro 4,7-méthanoisobenzofuran-1-one, racémique ou optiquement active, le composé de formule III est le menthol racémique ou optiquement actif et la séparation éventuelle des composés diastéréoisomères est effectuée par chromatographie ainsi qu'un procédé de préparation des composés de formule générale I, tels que définis précédemment, caractérisé en ce que le composé de formule II est la lactone d'un acide cis 2,2-diméthyl 3-(dihydroxyméthyl) cyclopropane-1-carboxylique, racémique ou optiquement actif, le composé de formule III est le menthol racémique ou optiquement actif et la séparation éventuelle des composés diastéréoisomères est effectuée par chromatographie.

L'invention a également pour objet l'application des composés de formule générale (I) au dédoublement des composés de formule (II) ou (III), application qui consiste à faire réagir un composé lactonique de formule générale (II)

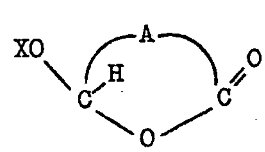

(II)

formule dans laquelle X représente un atome d'hydrogène ou un radical alcoyle comportant de 1 à 4 atomes de carbone et A conserve les significations précitées, en présence d'un acide, avec un alcool ou un phénol substitué de formule générale (III) ZOH (III)

formule dans laquelle Z conserve les significations précitées, pour obtenir ou bien un mélange de diastéréoisomères, dénommé ($I_B$), lorsque la lactone est un isomère optique bien défini et les centres chiraux de l'alcool ou du phénol ne sont pas tous de configuration univoque, ou bien un mélange de diastéréoisomères, dénommés ($I_C$) lorsque l'alcool ou le phénol est un isomère optique bien défini et les atomes chiraux de la lactone ne sont pas tous de configuration univoque, à séparer par une méthode

physique les éthers diastéréoisomères contenus ou bien dans les mélanges du type ($I_B$) ou bien dans les mélanges du type ($I_C$) et, notamment, l'éther dénommé ($I_A$), dont les centres chiraux sont tous de configuration univoque et caractérisée en ce que l'on soumet chacun des éthers diastéréoisomères ainsi séparés, à une hydrolyse ou à une alcoolyse, en milieu acide, pour obtenir soit un composé de type II et les autres diastéréoisomères provenant éventuellement de l'existence de plusieurs centres asymétriques, soit un composé de type III et les autres diastéréoisomères provenant éventuellement de l'existence de plusieurs centres asymétriques, ces composés II et III et les divers diastéréoisomères correspondants, comportant des centres chiraux de configuration univoque, autrement dit des centres chiraux dédoublés par rapport aux centres chiraux correspondants des alcools ou des phénols de départ, ou par rapport aux centres chiraux de composés lactoniques de départ.

La solvolyse acide des composés du type $I_A$ (diastéréoisomères de configuration univoque), qu'ils soient issus des mélanges de diastéréoisomères ($I_B$) ou ($I_C$), permet d'obtenir l'alcool ou le phénol ZOH (III) ainsi que la lactone (II), chacun sous forme d'un isomère optique, c'est-à-dire dont toutes les configurations des centres chiraux sont parfaitement définies, soit (R), soit (S).

Par application de la méthode telle que décrite au schéma (III) dans lequel, au départ, l'alcool ne comportant qu'un seul centre chiral est un racémate formé de 2 antipodes (R) et (S), on peut, en lui opposant un isomère optique bien défini de la lactone (II), obtenir deux éthers du type ($I_A$) appelés respectivement ($I_D$) et ($I_E$) que l'on peut séparer par un traitement physique, notamment, par cristallisation ou chromatographie. Dans ces deux composés ($I_D$) et ($I_E$), le reste alcoolique présente une configuration antipodale. Par solvolyse acide soit de ($I_D$) soit de ($I_E$), on récupère ainsi l'alcool ZOH (III), dédoublé en ses antipodes (R) et (S), respectivement (cf. schéma III).

Par application de la méthode décrite au schéma (IV), en utilisant au départ une lactone dont les centres chiraux présentent des configurations correspondant à un racémate et en lui opposant un isomère optique parfaitement défini d'un alcool ou d'un phénol (III), on peut également obtenir 2 éthers de type ($I_A$), appelés respectivement ($I_F$) et ($I_G$), que l'on peut séparer par une méthode physique, notamment, par chromatographie ou cristallisation.

Dans ces deux composés ($I_F$) et ($I_G$), le reste lactonique présente une configuration antipodale. Par solvolyse acide soit de ($I_F$), soit de ($I_G$) on récupère ainsi la lactone (II) dédoublée en ses antipodes (cf. schéma IV).

L'invention a plus particulièrement pour objet l'application telle que définie précédemment qui consiste à faire réagir la lactone de l'acide cis 2,2-diméthyl 3S-(dihydroxyméthyl) cyclopropane-1R-carboxylique, en présence d'un agent acide, avec la 1(RS) - hydroxy 2 - méthyl 3 - (2 - propen - 1 - yl) cyclopent - 2 - èn - 4 - one, à séparer par cristallisation dans l'isopropanol le (1R,5S) 6,6 - diméthyl 4(R) - 1/(R) - 2 - méthyl 4 - oxo 3 - (2 - propen - 1 - yl) cyclopent - 2 - enyloxy)/ 3 - oxa bicyclo (3.1.0) hexan - 2 - one du (1R,5S) 6,6 - diméthyl 4(R) /1(S) - 2 - méthyl 4 - oxo 3 - (2 - propen - 1 - yl) cyclopent - 2 - enyloxy) /3 - oxa bicyclo (3.1.0.) hexan - 2 - one, et est caractérisée en ce que l'on soumet ce dernier isomère « 1S » à une solvolyse en milieu acide pour obtenir la 1(S) - hydroxy 2 - méthyl 3 - (2 - propen - 1 - yl) cyclopent - 2 - èn 4 - one.

Bien entendu, la solvolyse de l'isomère « 1R » permet d'obtenir la 1(R) - hydroxy 2 - méthyl 3 - (2 - propen - 1 - yl) cyclopent - 2 - èn 4 - one.

Dans cette application la solvolyse est effectuée avantageusement en milieu auqueux, en présence d'acide chlorhydrique, ou bien dans le méthanol, en présence d'acide paratoluène sulfonique. L'invention a également pour objet l'application telle que définie précédemment qui consiste à faire réagir la lactone de l'acide dl cis 2,2 - diméthyl 3 - (dihydroxy méthy) cyclopropane 1 - carboxylique, en présence d'un agent acide avec la 1(S) - hydroxy 2 - méthyl 3 - (2 - propen - 1 - yl) cyclopent - 2 - èn 4 - one, à séparer par cristallisation dans l'éther isopropylique le (1R, 5S) 6,6 - diméthyl 4(S)/1(S) 2 - méthyl 4 - oxo 3 - (2 - propèn - 1 - yl) cyclopent - 2 - enyloxy/3 - oxa bicyclo (3.1.0) hexan - 2 - one du (1R, 5S) 6,6 - diméthyl 4(R)/1(S) 2 - méthyl 4 - oxo 3 - (2 - propèn - 1 - yl) cyclopent - 2 - enyloxy/3 - oxa bicyclo (3.1.0) hexan - 2 - one, étant caractérisée en ce que l'on soumet ce dernier isomère (4S) à une solvolyse en milieu acide pour obtenir la lactone de l'acide cis 2,2 - diméthyl 3S - (dihydroxyméthyl) cyclopropane - 1R - carboxylique.

Dans cette application, l'agent acide utilisé est notamment l'acide chlorhydrique.

L'invention a également pour objet l'application telle que définie précédemment qui consiste à faire réagir la lactone de l'acide cis 2,2 - diméthyl 3 - S (dihydroxyméthyl) cyclopropane - 1R - carboxylique, en présence d'un agent acide, avec la 2 (R,S) hydroxy 3,3 - diméthyl butyrolactone, à séparer par chromatographie le (1R, 5S) 6,6 - diméthyl 4(R)/3',3' - diméthylbutyrolactone 2' - (S) - oxy/3 - oxa bicyclo /3.1.0/ hexan - 2 - one du (1R, 5S) 6,6 - diméthyl 4(R) - /3',3' - diméthyl butyrolactone 2'(R) oxy/3 - oxa bicyclo /3.1.0/ hexan - 2 - one, et est caractérisée en ce que l'on soumet l'un ou l'autre de ces isomères à une solvolyse, en milieu acide, pour obtenir la 2(S) - hydroxy 3,3 - diméthyl butyrolactone ou la 2(R) - hydroxy 3,3 - diméthyl butyrolactone selon l'isomère choisi.

Bien entendu, l'isomère « 2'R » de l'éther conduit la 2(R) - hydroxy 3,3 - diméthyl butyrolactone, et l'isomère « 2'S » à la 2(S) - hydroxy 3,3 - diméthyl butyrolactone.

Dans cette application, la solvolyse peut être effectuée, par exemple, par l'acide paratoluène sulfonique en milieu aqueux ou dans le méthanol.

L'invention a également pour objet l'application telle que définie précédemment, qui consiste à faire réagir la (3R) (3aR) (4S) (7R) (7aS) 3 - hydroxy tétrahydro 4,7-méthano isobenzofuran-1-one avec le (R,S)

menthol, en présence d'un agent acide, à séparer par chromatographie la (3R) (3aR) (4S) (7R) (7aS) 3 - /(1'R) (2'R) (5'R), 2' - isopropyl 5' - méthylcyclohexanoxy/tétrahydro 4,7 - méthanoisobenzofuran - 1 - one de la (3R) (3aR) (4S) (7R) (7aS) 3-/(1'S) (2'R) (5'S) 2'-isopropyl 5'-méthyl cyclohexan-oxy/-tétrahydro 4,7-méthanoisobenzofuran - 1 - one, et est caractérisée en ce que l'on soumet l'un ou l'autre de ces isomères à une solvolyse en milieu acide pour obtenir le (R) ou le (S)-menthol.

L'invention a également pour objet l'application telle que définie précédemment, qui consiste à faire réagir la lactone de l'acide cis 2,2 - diméthyl 3S - (dihydroxyméthyl) cyclopropane - 1R - carboxylique, en présence d'un agent acide, avec le (R, S) menthol, à séparer par chromatographie la (1R, 5S) 6,6 - diméthyl (4R)/(1'R) (2'S) (5'R) 2' - isopropyl 5' - méthylcyclohexanoxy/3 - oxa bicyclo /3.1.0/ hexan - 2 - one de la (1R, 5S) 6,6 - diméthyl 4R/(1'S) (2'R) (5'S) 2' - isopropyl 5' - méthyl cyclohexanoxy/3 - oxa bicyclo /3.1.0/ hexan - 2 - one, et est caractérisée en ce que l'on soumet l'un ou l'autre de ces isomères à une solvolyse en milieu acide pour obtenir le (R) ou le (S)-menthol.

Dans cette application et la précédente, la solvolyse peut être effectuée par exemple, par l'acide paratoluène sulfonique en milieu aqueux.

On connaissait déjà quelques procédés généraux de dédoublement d'alcools comportant des carbones asymétriques. Par exemple, on savait dédoubler certains alcools racémiques (R, S), en les combinant avec un acide organique optiquement actif, en séparant, par un traitement physique convenable, l'ester d'alcool (S) et l'ester d'alcool (R) résultants puis en hydrolysant, séparément, ces deux esters pour obtenir les alcools de structure (R) ou de structure (S).

On connaissait également un procédé plus compliqué consistant à dédoubler des alcools racémiques (RS) en les combinant à un diacide organique, en faisant réagir l'hémiester résultant, avec une base optiquement active, pour obtenir les sels diastéréoisomères correspondants, à acidifier pour libérer les hémiesters d'alcool (S) et d'alcool (R) puis à hydrolyser, séparément, ces hémiesters pour obtenir l'alcool de structure (S) et l'alcool de structure (R).

En fait, ce type de dédoublement ne s'applique sans difficulté qu'aux alcools suffisamment stables, insensibles ou peu sensibles aux conditions de formation et d'hydrolyse des esters ou hémiesters intermédiaires.

La société demanderesse, a maintenant, selon la présente invention, mis au point une nouvelle méthode, très générale, permettant de dédoubler des alcools ou des phénols substitués ZOH (III), comportant un ou plusieurs atomes de carbone asymétrique, méthode qui ne comporte pas les inconvénients des méthodes citées précédemment.

Cette nouvelle méthode dont les diverses étapes ont été décrites ci-dessus, consiste à faire réagir, en présence d'un agent acide, l'alcool ou le phénol substitué ZOH de formule (III), comportant au moins un atome de carbone asymétrique de configuration racémique (RS) avec un isomère optique d'une lactone (II) possédant un ou plusieurs atomes chiraux de configuration stérique parfaitement définie, soit (R), soit (S), pour obtenir un mélange équimoléculaire de diastéréoisomères du type ($I_A$), dans chacun desquels le reste (Z) possède une stéréochimie univoque au niveau de chaque centre chiral. On sépare ensuite les diastéréoisomères du type ($I_A$) par des moyens physiques tels qu'une chromatographie ou une cristallisation dans un solvant, soumet individuellement ces composés diastéréoisomères de type ($I_A$) à une solvolyse en milieu acide, pour obtenir respectivement l'alcool ou le phénol substitué ZOH (III) de stéréochimie univoque.

Cette méthode présente notamment l'avantage sur les méthodes de dédoublement déjà existantes, d'être d'une grande simplicité puisqu'elle ne comporte que les étapes suivantes : combinaison des alcools ou des phénols ZOH (III), avec le composé (II), celui-ci étant pris sous forme d'un isomère optique de stéréochimie déterminée, pour obtenir les éthers diastéréoisomères correspondants ($I_B$), séparation par un moyen physique des diastéréoisomères contenus en composés du type $I_A$, comportant une stéréochimie bien définie, puis solvolyse de chaque composé du type ($I_A$) pour obtenir les composés ZOH (III) dédoublés.

La méthode de dédoublement selon l'invention présente, par ailleurs, une grande généralité d'application. La combinaison du composé ZOH (III) et d'un isomère optique (II), est effectuée avec un bon rendement. Les éthers en résultant possèdent, en général, une structure telle que la séparation par un moyen physique, de stéréoisomères du type $I_A$ est aisée. Cette séparation peut souvent être effectuée par cristallisation dans un solvant convenable. La solvolyse des diastéréoisomères du type ($I_A$), en milieu acide, conduit sans difficulté et avec de bons rendements, aux alcools ou phénols dédoublés attendus.

On ne décèle pas, lors de cette solvolyse finale, d'altération notable des composés ZOH, (III). La stéréochimie des composés (II) n'est maintenue, lors de la solvolyse, que si la structure de la molécule (II) impose à l'hydroxyle une disposition univoque.

C'est ainsi que la méthode de dédoublement selon l'invention s'applique notamment à ces alcools spécialement fragiles que sont les cyanhydrines.

Une telle méthode de dédoublement des alcools ou des phénols substitués, de portée générale, est très utile car elle permet d'obtenir dans la majorité des cas et dans des conditions avantageuses, à partir de composés racémiques obtenus par synthèse chimique, les isomères optiques dédoublés dont l'un d'entre eux détient, en général, la quasi-totalité de l'activité de la molécule concernée, (cas des composés naturels qui sont optiquement actifs).

Des données précédentes, il ressort que la société demanderesse a également mis au point une

nouvelle méthode de dédoublement de composés lactoniques (II) qui comportent un ou plusieurs atomes chiraux. Cette nouvelle méthode consiste à faire réagir, en présence d'un agent acide, le composé lactonique (II) comportant au moins un atome chiral de configuration racémique (RS), avec un isomère optique d'un alcool ou d'un phénol substitué de formule (III) de configuration stérique déterminée (R) ou (S), pour obtenir un mélange d'éthers diastéréoisomères dénommé ($I_C$), à soumettre ce mélange à un traitement physique tel qu'une chromatographie ou une cristallisation pour séparer les composés diastéréoisomères du type ($I_A$) contenus dans ce mélange, chaque diastéréoisomère présentant dans sa partie lactonique une stéréochimie bien définie, puis à hydrolyser ces composés en milieu acide pour obtenir le composé (II) sous forme dédoublée.

Une telle méthode n'avait jamais, jusqu'ici, été décrite dans la littérature.

Elle présente, d'une manière générale, les mêmes avantages et la même simplicité que la méthode de dédoublement des alcools et des phénols décrits précédemment.

Les exemples suivants illustrent l'invention sans la limiter.

Exemple 1 : Mélange de (1R, 5S) 6,6 - diméthyl 4(R)/1(S) - méthyl 4 - oxo 3 - (2 - propen - 1 - yl) cyclopent - 2 - enyloxy/3 - oxa bicyclo (3.1.0) hexan - 2 - one et de (1R, 5S) 6,6 - diméthyl 4(R)/1(R) - 2 - méthyl 4 - oxo 3 - (2 - propen - 1 - yl) cyclopent - 2 - enyl - oxy 3 - oxa bicyclo (3.1.0) hexan - 2 - one à partir de 1(R) - hydroxy - 2 méthyl 3 - (2' - propen - 1 - yl) cyclopent - 2 - ène 4 - one.

Dans 1 000 $cm^3$ de benzène on introduit 152 g de 1 (RS) - hydroxy 2 - méthyl 3 - (2 - propen - 1 - yl) cyclopent - 2 - èn 4 - one, 152 g de lactone de l'acide cis 2,2 - diméthyl 3S - (dihydroxy méthyl) cyclopropane - 1R - carboxylique, 3,5 g d'acide paratoluène sulfonique, porte le mélange réactionnel au reflux, maintient le reflux pendant 4 heures en séparant, par décantation azéotropique l'eau formée, laisse refroidir à 20 °C, amène à pH 7-8 par addition de triéthylamine, concentre à sec par distillation sous pression réduite et obtient un mélange (1R, 5S) 6,6 - diméthyl 4(R)/1(S) - 2 - méthyl 4 - oxo 3 - (2 - propen - 1 - yl cyclopent - 2 - enyloxy/3 - oxa bicyclo (3.1.0) hexan - 2 - one et de (1R, 5S) 6,6 - diméthyl 4(R)/1(R) - 2 - méthyl 4 - oxo 3 - (2 - propen - 1 - yl) cyclopent - 2 - enyloxy/3 - oxa bicyclo (3.1.0) hexan - 2 - one.

Exemple 2 : (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one, à partir d'un mélange de (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one et de (1R, 5S) 6,6 - diméthyl 4(R)/1(R) - 2 - méthyl 4 - oxo 3 - (2 - propen - 1 - yl) cyclopent - 2 - enyloxy/3 - oxa bicyclo (3.1.0) hexan - 2 - one.

On cristallise le mélange de (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one et de (1R, 5S) 6,6 - diméthyl 4(R)/1(R) - 2 - méthyl 4 - oxo 3 - (2 - propen - 1 - yl) cyclopent - 2 - enyloxy 3 - oxabicyclo (3.1.0) hexan - 2 - one obtenu à l'exemple 1, dans 300 $cm^3$ d'isopropanol, refroidit à 0 °C, agite, isole par essorage le précité formé, le lave, le sèche et obtient 82,6 g de (1R, 5S) 6,6 - diméthyl 4(R)/1(S) - 2 - méthyl 4 - oxo - 3 - (2 - propen - 1 - yl) cyclopent - 2 - enyl oxy/3 - oxa bicyclo (3.1.0) hexan - 2 - one, F = 104 °C.

$(\alpha)^{20}_D = -66,5°$ (c = 1,1 %, benzène)

Analyse : $C_{16}H_{20}O_4$ (276)

| | C % | H % |
|---|---|---|
| Calculés | 69,54 | 7,3 |
| Trouvés | 69,3 | 7,4 |

Dichroïsme circulaire (dioxane)

$\Delta\varepsilon = -26,4$ à 222 nm (max.) ; $\Delta\varepsilon = +2,80$ à 321 nm (max.) ;

$\Delta\varepsilon = +2,50$ à 332 nm (max.)

Spectre de R.M.N. (deutérochloroforme)

Pic à 1,22 ppm caractéristique de l'hydrogène des méthyles géminés, pics de 1,97 à 2,33 ppm caractéristiques des hydrogènes du cycle cyclopropanique ; pic à 2,1 ppm caractéristique des hydrogènes du méthyl en position 2 de l'alléthrolone.

Pics à 2,92-3,02 ppm caractéristiques du méthylène en position 1 de la chaîne allylique de l'alléthrolone ; pic à 4,63 ppm caractéristique de l'hydrogène en position 1 de l'alléthrolone ; pics à 4,75-5,17 ppm, caractéristiques des hydrogènes du méthylène terminal en position 3 de la chaîne allylique de l'alléthrolone ; pic à 5,33 ppm, caractéristique de l'hydrogène porté par le carbone en $\alpha$ de l'oxygène endocyclique ; pics à 5,47-6,16 ppm caractéristiques de l'hydrogène en position 2 de la chaîne allylique de l'alléthrolone.

Exemple 3 : (1R, 5S) 6,6 - diméthyl 4(R)/1(R) - 2 - méthyl 4 - oxo 3 - (2 - propen - 1 - yl) cyclopent - 2 - enyloxy/ 3 - oxa bicyclo (3.1.0) hexan - 2 - one à partir de 1(R) - hydroxy 2 - méthyl 3 - (2 - propen - 1 - yl) cyclopent - 2 - èn 4 - one/ou (R) alléthrolone/.

Dans 75 $cm^3$ de benzène on introduit 7,5 g de lactone de l'acide cis 2,2 - diméthyl 3S - (dihydroxy, méthyl) cyclopropane - 1R - carboxylique, 7,5 g de 1(R) - hydroxy 2 - méthyl 3 - (2 - propen - 1 - yl) cyclopent - 2 - ène 4 - one, 0,100 g d'acide paratoluène sulfonique, porte le mélange réactionnel au reflux, maintient le reflux pendant 4 heures en séparant, par décantation azéotropique, l'eau formée, concentre à sec par distillation sous pression réduite et obtient 15 g de (1R, 5S) 6,6 - diméthyl 4(R)/1(R) 2 - méthyl 4 - oxo 3 - (2 - propen - 1 - yl) cyclopent - 2 - enyloxy/3 - oxa bicyclo (3.1.0) hexan 2 - one.
Dichroïsme circulaire (dioxane)
$\Delta\varepsilon = -2,56$ à 330 nm (max.) ; $\Delta\varepsilon = -2,8$ à 316 nm (max.) ; $\Delta\varepsilon = +14,6$ à 225 nm (max.).
Spectres de R.M.N. (deuterochloroforme)

Pics à 1,22-1,23 ppm caractéristiques des hydrogènes des méthyles géminés ; pic à 2,07 ppm caractéristiques des hydrogènes du méthyle en position 2 de l'alléthrolone ; pics à 2,92-3,02 ppm, caractéristiques des hydrogènes du méthylène en position 1 de la chaîne allylique de l'alléthrolone ; pics à 4,83-5,08 ppm, caractéristiques des hydrogènes du méthylène en position 3 de la chaîne allylique de l'alléthrolone ; pic à 4,87 ppm, caractéristique de l'hydrogène en position 1 de l'alléthrolone ; pic à 5,26 ppm caractéristique de l'hydrogène porté par le carbone en $\alpha$ de l'oxygène endocyclique ; pics à 5,47-6,33 ppm caractéristique de l'hydrogène en position 2 de la chaîne allylique de l'alléthrolone.

Exemple 4 : (1R, 5S) 6,6 - diméthyl 4(R)/1(S) - 2 - méthyl 4 - oxo 3 - (2 - propen - 1 - yl) cyclopent - 2 - enyloxy/ 3 - oxa bicyclo (3.1.0) hexan 2 - one, à partir de 1(S) - hydroxy 2 - méthyl 3 - (propen - 1 - yl) cyclopent - 2 - èn 4 - one/ou (S) - alléthrolone/.

Dans un mélange de 30 $cm^3$ d'éther de pétrole (éb. = 35 - 37 °C) et de 3 $cm^3$ de benzène on introduit 5 g de lactone de l'acide cis 2,2 - diméthyl 3S - (dihydroxy - méthyl) cyclopropane - 1R - carboxylique 5 g de 1(S) hydroxy 2 - méthyl 3 - (2 - propen - 1 - yl) cyclopent - 2 - ène 4 - one, 0,05 g d'acide paratoluène sulfonique, porte le mélange réactionnel au reflux, sous agitation, agite pendant 4 heures au reflux en séparant, par décantation azéotropique l'eau formée, refroidit à 0 °C, agite, isole par essorage le précipité formé, le lave, le sèche et obtient 8,7 g de (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one, F = 104 °C.

Les liqueurs mères sont concentrées à sec, on cristallise le résidu dans l'isopropanol et obtient 0,12 g de deuxième jet.

Exemple 5 : (1R, 5S) 6,6 - diméthyl 4(R)/1(S) - 2 - méthyl 4 - oxo 3 - (2 - propen - 1 - yl) cyclopent - 2 - enyloxy) 3 - oxa bicyclo (3.1.0) hexan - 2 - one à partir de la lactone de l'acide dl cis 2,2 - diméthyl 3 - (dihydroxyméthyl) cyclopropane - 1 - carboxylique.

Dans un récipient surmonté d'un système de décantation azéotropique, on introduit 25,2 g de 1(S) - hydroxy 2 - méthyl 3 - (2 - propen - 1 - yl) cyclopent - 2 - ène 4 - one (ou (S) alléthrolone), 250 $cm^3$ de benzène, 23,5 g de lactone de l'acide d1 cis 2,2 - diméthyl 3 - (dihydroxyméthyl) cyclo propan - 1 - carboxylique et 0,25 g d'acide paratoluène sulfonique, porte le mélange réactionnel au reflux sous agitation, en éliminant, par décantation azéotropique, l'eau formée, agite ainsi pendant 4 heures, refroidit, neutralise par addition de triéthylamine, concentre à sec sous vide, ajoute au résidu 5 $cm^3$ d'éther isopropylique, agite à 0 °C pendant une heure, isole par essorage le précité formé, le lave, le sèche et obtient 15,1 g de (1R, 5S) 6,6 - diméthyl 4(R)/1(S) 2 - méthyl 4 - oxo 3 - (2 - propen - 1 - yl) cyclopent - 2 - enyloxy) 3 - oxa bicyclo (3.1.0) hexan - 2 - one. F = 104 °C.

Exemple 6 : 1(S) - hydroxy 2 - méthyl 3 - (2 - propen - 1 - yl) cyclopent - 2 - ène 4 - one/ ou (S) alléthrolone/ à partir de (1R, 5S) 6,6 - diméthyl 4(R)/1(S) 2 - méthyl 4 - oxo 3 - (2 - propen - 1 - yl) cyclopent - 2 - enyloxy/3 - oxa bicyclo (3.1.0) hexan - 2 - one.

On mélange 82,6 g de (1R, 5S) 6,6 - diméthyl 4(R)/1(S) - 2 - méthyl 4 - oxo 3 - (2 - propen - 1 - yl) cyclopent - 2 - enyloxy/ 3 - oxa bicyclo (3.1.0) hexan - 2 - one, obtenu à l'exemple 4, 826 $cm^3$ d'eau, 8,2 $cm^3$ de solution aqueuse d'acide chlorhydrique à 22 °Bé, agite pendant 72 heures à 20 °C, amène à pH 10,5 la solution obtenue par addition de solution aqueuse normale de soude, extrait au chlorure de méthylène, sèche, concentre à sec, par distillation sous pression réduite et obtient 42,9 g de 1(S) - hydroxy 2 - méthyl 3 - (2 - propen - 1 - yl) cyclopent - 2 - ène 4 - one/ ou (S) alléthrolone/.
$(\alpha)^{20}_D = +14°$ (c = 1,2 %, chloroforme).
Dichroïsme circulaire (dioxane)
$\Delta\varepsilon = -20$ à 230 nm (max.) ; $\Delta\varepsilon = +3,36$ à 321 nm (max.) ; $\Delta\varepsilon = +3,0$ à 331 nm (max.).

Exemple 7 : 1(R) - hydroxy 2 - méthyl 3 - (2 - propen - 1 - yl) cyclopent - 2 - ène 4 - one/ ou (R) alléthrolone/, à partir de (1R, 5S) 6,6 - diméthyl 4(R)/1(R) - 2 - méthyl 4 - oxo 3 - (2 - propen - 1 - yl) cyclopent - 2 - enyloxy/3 - oxa bicyclo (3.1.0) hexan - 2 - one.

On mélange 82,4 g de (1R, 5S) 6,6 - diméthyl 4(R)/1(R) - 2 - méthyl 4 - oxo 3 - (2 - propen - 1 - yl - cyclopent - 2 - enyloxy/3 - oxa bicyclo (3.1.0) hexan - 2 - one, obtenus à l'exemple 3, 824 $cm^3$ d'eau,

8,2 $cm^3$ de solution aqueuse d'acide chlorhydrique à 22 °Bé, agite pendant 72 heures à 20 °C, amène à pH 10,5 la solution obtenue par addition de solution aqueuse normale de soude, extrait au chlorure de méthylène, sèche, concentre à sec par distillation sous pression réduite et obtient 42,7 g de 1(R) - hydroxy 2 - méthyl 3 - (2 - propen - 1 - yl) cyclopent - 2 - ène 4 - one/ ou (R) alléthrolone/.
$(\alpha)_D = -14,5$ °C (c = 1,2 %, chloroforme).
Dichroïsme circulaire (dioxane)
$\Delta\varepsilon = +20$ à 230 nm (max.) ; $\Delta\varepsilon = -3,36$ à 321 nm (max.) ; $\Delta\varepsilon = -3,0$ à 331 nm (max.).

Exemple 8 : 1(S) - hydroxy 2 - méthyl 3 - (2 - propen - 1 - yl) cyclopent - 2 - ène 4 - one/ ou (S) alléthrolone/, à partir de (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one.

Dans 42 $cm^3$ de méthanol, on introduit 0,83 g d'acide paratoluène sulfonique, 8,3 g de (1R, 5S) 6,6 - diméthyl 4(R)/1(S) - 2 - méthyl 4 - oxo 3 - (2 - propen - 1 - yl) cylcopent - 2 - enyloxy/3 - oxa bicyclo (3.1.0) hexan - 2 - one, obtenu à l'exemple 2, agite pendant 2 heures à 20 °C, amène le mélange réactionnel à pH 7 par addition de carbonate de sodium, filtre, concentre le filtrat sous pression réduite, chromatographie le résidu (10 g) sur gel de silice, en éluant avec un mélange de benzène et d'acétate d'éthyl (7/3) et obtient 4,4 g de 1(S) - hydroxy 2 - méthyl 3 - (2 - propen - 1 - yl) cyclopent - 2 - ène 4 - one/ ou (S) alléthrolone/.
$(\alpha)^{20}_D = +14,5$ ° (c = 1,2 %, chloroforme).
Dichroïsme circulaire (dioxane)
$\Delta\varepsilon = -20,4$ à 229 nm (max.) ; $\Delta\varepsilon = +3,36$ à 321 nm (max.) ; $\Delta\varepsilon = +3,08$ à 331 nm (max.).

Exemple 9 : Lactone de l'acide cis 2,2 - diméthyl 3S - (dihydroxyméthyl) cyclopropane - 1 - R carboxylique à partir de (1R, 5S) 6,6 - diméthyl 4(R)/1(S) - 2 - méthyl 4 - oxo 3 - (2 - propen - 1 - yl) cyclopent - 2 - ènyloxy/3 - oxa bicyclo (3.1.0) hexan - 2 - one.

Dans un mélange de 100 $cm^3$ d'eau et de 1 $cm^3$ de solution aqueuse d'acide chlorhydrique à 22 °Baumé, on introduit 2,76 g de (1R, 5S) 6,6 - diméthyl 4(R)/1(S) - 2 - méthyl 4 - oxo 3 - (2 - propen - 1 - yl) cyclopent - 2 - ènyloxy/3 - oxa bicyclo (3.1.0) hexan - 2 - one, F = 104 °C, obtenu à l'exemple 5, agite la suspension réactionnelle pendant 72 heures, amène le mélange réactionnel à pH 10, par addition de solution aqueuse normale de soude, extrait la solution aqueuse basique au chlorure de méthylène, lave la phase organique à l'eau, la sèche, la concentre à sec sous pression réduite et obtient 1,39 g de (S) alléthrolone.

On sature la phase aqueuse par du sulfate d'ammonium, l'acidifie par une solution aqueuse d'acide chlorhydrique, agite pendant une heure, sépare par essorage le produit gommeux insolubilisé, le dissout dans l'acétate d'éthyle, filtre, concentre le filtrat à sec et obtient 1,32 g de l'actone de l'acide cis 2,2 - diméthyl 3S - (dihydroxyméthyl) cyclopropane - 1R - carboxylique.

Exemple 10 : (3R, 3aR, 4S, 7R, 7aS) 3 -/(1R) 2 - méthyl 4 - oxo 3 - (2 - propen 1 - yl) cyclopent 2 - ényloxy/ tétrahydro 4,7 - méthano isobenzofuran 1 - one.

Dans 50 $cm^3$ de benzène anhydre, on introduit 4,3 g de (3R, 3aR, 4S, 7R, 7aS) 3 - hydroxy tétrahydro 4,7 - méthano isobenzofuran 1 - one décrit ci-après à l'exemple 24, 3,9 g de 1(R,S) - hydroxy 2 - méthyl 3 - (2 - propen - 1 - yl) cyclopent - 2 - ène 4 - one et, 40 mg d'acide paratoluène sulfonique. On porte le mélange réactionnel au reflux, maintient le reflux pendant 18 heures puis concentre à sec par distillation sous pression réduite. On chromatographie le résidu sur gel de silice en éluant avec un mélange chloroforme-acétone (100-2,5). On obtient 2,4 g de produit attendu sous forme d'une huile épaisse.

Exemple 11 : (3R, 3aR, 4S, 7R, 7aS) 3 -/(1S) 2 - méthyl 4 - oxo 3 - (2 - propen 1 - yl) cyclopent 2 - ényloxy/tétrahydro 4,7 méthano isobenzofuran 1 - one.

En poursuivant la chromatographie décrite à l'exemple 10, on recueille 2,7 g de produit attendu sous forme de cristaux blancs. F = 148 °C.

Exemple 12 : (3R, 3aR, 4S, 7R, 7aS) 3-/(1S) 2 - méthyl 4 - oxo 3 - (2 - propen 1 - yl) cyclopent 2 - ényloxy/tétrahydro 4,7 - méthano isobenzofuran 1 - one (isomère A) et (3S, 3aS, 4R, 7S, 7aR) 3 -/(1S) 2 - méthyl 4 - oxo 3 - (2 - propen 1 - yl) cyclopent 2 - ényloxy/tétrahydro 4,7 - méthano isobenzofuran 1 - one (isomère B).

Dans 200 $cm^3$ de benzène on introduit 33 g de 3 - hydroxy tétrahydro 4,7 - méthano isobenzofuran 1 - one racémique, 30 g de 1(S) hydroxy 2 - méthyl 3 - (2 - propen - 1 - yl) cyclopent 2 - ène 4 - one (ou (S) alléthrolone) et 0,18 g d'acide paratoluènesulfonique.

On chauffe au reflux pendant 4 heures puis concentre à sec par distillation sous pression réduite. Les 60,3 g de résidu obtenu sont chromatographiés sur silice, élués par un mélange chloroforme-acétone

(100-5). On obtient 17,4 g de (3R, 3aR, 4S, 7R, 7aS) 3-/(1S) 2 - méthyl 4 - oxo 3 - (2 - propen 1 - yl) cyclopent 2 - ényloxy/ tétrahydro 4,7 - méthano isobenzofuran 1 - one (isomère A) et 13,6 g de (3S, 3aS, 4R, 7S, 7aR) 3-/(1S) 2 - méthyl 4 - oxo 3 - (2 - propen 1 - yl) cyclopent 2 - ényloxy/tétrahydro 4,7 méthano isobenzofuran 1 - one (isomère B).

L'isomère A présente les caractéristiques suivantes : F = 148 °C.
$/\alpha/^{20}_D = -8°$ (C = 1,5 % benzène)
Analyse : $C_{18}H_{20}O_4$ : 300,36

| | C % | H % |
|---|---|---|
| Calculés | 71,98 | 6,71 |
| Trouvés | 72,0 | 6,8 |

Spectre I.R. (chloroforme)
Absorption à 1775 $cm^{-1}$ caractéristique de la lactone,
Absorption à 1700 $cm^{-1}$ caractéristique du carbonyle,
Absorption à 1657 $cm^{-1}$, 1640 $cm^{-1}$ caractéristique de la double liaison éthylènique conjuguée,
Absorption à 981-918 $cm^{-1}$ caractéristique du vinyle.
Dichroïsme circulaire (dioxane)
$\Delta\varepsilon = -18,3$ à 225 nm (max.) ;
$\Delta\varepsilon = +2,29$ à 321 nm (max.) ;
$\Delta\varepsilon = +2,59$ à 332 nm (max.).
Spectre R.M.N. (CD $Cl_3$)
Pics à 6,25-6,28 p.p.m. caractéristiques des hydrogènes éthylèniques de la lactone (en 5,6).
Pic à 5,1 p.p.m. caractéristique de l'hydrogène en position 3 de la lactone.
Pics de 1,33 à 1,75 p.p.m. caractéristiques des hydrogènes du $CH_2$ de la lactone (en 8).
Pic à 4,58 p.p.m. caractéristique de l'hydrogène en 1 du cycle alléthrolone.
Pic à 2,07 p.p.m. caractéristique des hydrogènes du méthyle en 2 du cycle alléthrolone.
Pics de 5,17 à 6,33 p.p.m. caractéristiques des hydrogènes en 2' du propényle.
Pics de 4,83 à 5,13 p.p.m. caractéristiques des hydrogènes en 3' du propényle.

L'isomère B présente les caractéristiques suivantes :
$/\alpha/^{20}_D = +42,5°$ (C = 1 %, benzène)
Analyse : $C_{18}H_{20}O_4$ : 300,36

| | C % | H % |
|---|---|---|
| Calculés | 71,98 | 6,71 |
| Trouvés | 71,8 | 6,7 |

Spectre I.R. (chloroforme)
Absorption à 1770 $cm^{-1}$ caractéristique du carbonyle,
Absorption à 1706 $cm^{-1}$ caractéristique du carbonyle conjugué
Absorption à 1656-1640 $cm^{-1}$ caractéristique de la double liaison éthylènique conjuguée,
Absorption à 982-918 $cm^{-1}$ caractéristique du groupement vinyle.
Dichroïsme circulaire (dioxane)

$\Delta\ \varepsilon = -18,4$ à 226 nm (max.)
$\Delta\ \varepsilon = +\ 2,9$ à 319 nm (max.)
$\Delta\ \varepsilon = +\ 2,63$ à 331 nm (max.)

Spectres R.M.N. ($CDCl_3$)
Pics à 6,25-6,28 p.p.m. caractéristiques des hydrogènes éthyléniques de la lactone (en 5-6).
Pic à 5,0 p.p.m. caractéristique de l'hydrogène en position 3 de la lactone.
Pics de 1,33 à 1,75 p.p.m. caractéristiques des hydrogènes du $CH_2$ de la lactone (en 8).
Pic à 4,73 p.p.m. caractéristique de l'hydrogène en position 1 du cycle alléthrolone.
Pic à 2,03 p.p.m. caractéristique des hydrogènes du méthyle en 2 sur l'alléthrolone.
Pics à 5,33-6,33 p.p.m. caractéristiques de l'hydrogène en 2' du propényle.
Pics de 4,83 à 5,13 p.p.m. caractéristiques des hydrogènes en 3' du propényle.

La 3-hydroxy tétrahydro 4,7-méthano isobenzofuran 1-one utilisée au départ de l'exemple a été préparée comme suit.

On agite à 20 °C sous atmosphère inerte 30 g de 5-hydroxy 2(5H) furanone, 150 $cm^3$ de chloroforme, 50 mg d'hydroquinone et 35 $cm^3$ de cyclopentadiène fraîchement distillé. On laisse 17 heures sous agitation en maintenant la température entre 20 et 45 °C puis concentre à sec sous pression réduite. Le résidu est cristallisé dans un mélange éther isopropylique-éther de pétrole.

On obtient 46,6 g de produit F = 103 °C.

Analyse : $C_9H_{10}O_3$ : 166,17

| | C % | H % |
|---|---|---|
| Calculé | 65,05 | 6,06 |
| Trouvé | 65,3 | 6,2 |

Spectre I.R. ($CHCl_3$)
OH : 3580 $cm^{-1}$
C = O 1770 $cm^{-1}$

Spectre R.M.N. ($CDCl_3$)
Pics à 6,2-6,22 p.p.m. caractéristiques des hydrogènes éthylèniques.
Pics à 5,18-5,27 p.p.m. caractéristiques de l'hydrogène en 1.
Pics à 4,87-4,95 p.p.m. caractéristiques de l'hydrogène de l'hydroxyle en 1.
Pics de 1,33 à 1,75 p.p.m. caractéristiques des hydrogènes des méthylènes.

Exemple 13 : (3S, 3aS, 4R, 7S, 7aR) 3-/(1R) 2 - méthyl 4 - oxo 3 - (2 - propen 1 - yl) cyclopent 2 - ényloxy/tétrahydro 4,7 - méthano isobenzofuran 1 - one (isomère D) et (3R, 3aR, 4S, 7R, 7aS) 3-/(1R) 2 - méthyl 4 - oxo 3 - (2 - propen 1 - yl) cyclopent 2 - ényloxy/tétrahydro 4,7 - méthano isobenzofuran 1 - one (isomère C).

Dans 100 $cm^3$ de benzène anhydre, on introduit 18,4 g de 3 - hydroxy tétrahydro 4,7 - méthano isobenzofuran 1 - one racémique préparé comme à l'exemple 12, 16 g de 1(R) hydroxy 2 - méthyl 3 - (2 - propen 1 - yl) cyclopent 2 - ène 4 - one (ou R alléthrolone) et 0,10 g d'acide paratoluène sulfonique monohydraté. On porte au reflux pendant 18 heures puis on chasse le solvant sous pression réduite. On obtient 31,4 g de produit brut que l'on chromatographie sur silice en éluant avec un mélange chloroforme-acétone (100-5). On recueille 9,4 g de (3S, 3aS, 4R, 7S, 7aR) 3-/(1R) 2 - méthyl 4 - oxo 3 - (2 - propen 1 - yl) cylcopent 2 - ényloxy/tétrahydro 4,7 méthano isobenzofuran 1 - one (isomère D) sous forme de cristaux blancs F = + 148 °C et 11,7 g de (3R, 3aR, 4S, 7R, 7as) 3-/ (1R) 2 - méthyl 4 - oxo 3 - (2 - propen - 1 - yl) cyclopent 2 - ényloxy/tétrahydro 4,7 - méthano isobenzofuran 1 - one (isomère C) sous forme de cristaux gommeux.

L'isomère D présente les caractéristiques suivantes :
$/\alpha/^{20}_D = + 11{,}5° \pm 1°$ (C = 1 % benzène)
Spectre R.M.N. ($CDCl_3$)
Pic à 6,23 p.p.m. caractéristique des hydrogènes éthyléniques de la lactone (en 5-6).
Pics à 1,33-1,75 p.p.m. caractéristiques des hydrogènes du $CH_2$ de la lactone en 8.
Pic à 5,0 p.p.m. caractéristique de l'hydrogène en position 3 de la copule lactonique.
Pics à 4,5-4,58 p.p.m. caractéristiques de l'hydrogène en position 1 du cycle de l'alléthrolone.
Pic à 2,06 p.p.m. caractéristique des hydrogènes du $CH_3$ de l'alléthrolone.
Spectre I.R. ($CHCl_3$)
C = O lactone 1775 $cm^{-1}$
C = O conjuguée 1700 $cm^{-1}$
C = C + conj. 1657-1640 $cm^{-1}$.
Dichroïsme circulaire (dioxane)
Accord avec éther de R alléthrolone, courbe pratiquement antipodale de celle de l'isomère A obtenu à l'exemple 16.
max. 225 nm $\Delta\varepsilon$ + 23,0
max. 320 nm $\Delta\varepsilon$ − 3,0
max. 332 nm $\Delta\varepsilon$ − 2,69

L'isomère C présente les caractéristiques suivantes :
$/\alpha/^{20}_D = - 51° \pm 1°$ (C = 1 % benzène)
Dichroïsme circulaire (dioxane)
En accord avec l'éther de R alléthrolone, courbe antipodale de celle de l'isomère B obtenu à l'exemple 12.
max. 224 nm $\Delta\varepsilon$ = + 19,1
max. 318 nm $\Delta\varepsilon$ = − 2,88
max. 330 nm $\Delta\varepsilon$ = − 2,59

Spectre R.M.N. ($CDCl_3$)
Pic à 6,26 p.p.m. caractéristique des hydrogènes éthyléniques de la copule lactonique.
Pics à 1,33-1,75 p.p.m. caractéristiques des hydrogènes du $CH_2$ de la copule lactonique.
Pic à 5,0 p.p.m. caractéristique de l'hydrogène en position 1 de la copule lactonique.
Pic à 4,7 p.p.m. caractéristique de l'hydrogène en position 1 du cycle de l'alléthrolone.

Pic à 2,0 p.p.m. caractéristique de l'hydrogène du $CH_3$ de l'alléthrolone.
Pics à 5,33-6,33 p.p.m. caractéristiques de l'hydrogène en position 2′ du propényle.
Pics à 4,83-5,1 p.p.m. caractéristiques de l'hydrogène en position 3′ du propényle.
Spectre I.R. ($CHCl_3$)
C = O lactone 1770 $cm^{-1}$
C = O conjuguée 1706 $cm^{-1}$
C = C + conjuguée 1656-1640 $cm^{-1}$

Exemple 14 : Le (1R, 5S) 6,6 - diméthyl 4(R) -/3′,3′ - diméthyl - butyrolactone 2′ - (S) oxy/3 - oxabicyclo /3.1.0/ hexan 2 - one (composé A) et le (1R, 5S) 6,6 - diméthyl 4(R) -/3′,3′ - diméthylbutyrolactone 2′(R) oxy /3 - oxabicyclo /3.1.0/ hexan 2 - one (composé B).

Sous atmosphère inerte on agite 7 g de pantolactone, 7,1 g de la lactone de l'acide 2,2 - diméthyl 3 - S (dihydroxyméthyl) cyclopropane 1R - carboxylique, 140 mg d'acide paratoluène sulfonique et 50 $cm^3$ de benzène. On chauffe 7 heures 15 minutes au reflux. Le benzène est distillé à 40 °C sous pression réduite. Le résidu est chromatographié sur silice, élué par un mélange benzène - acétate d'éthyle (85-15). On obtient 2,956 g de (1R, 5S) 6,6 - diméthyl 4 - (R) -/3′,3′ - diméthylbutyrolactone 2′ (S) - oxy/3 - oxabicyclo /3.1.0/ hexan 2 - one F = 102-104 °C (composé A) puis 547 mg de (1R, 5S) 6,6 - diméthyl 4-(S)/ 3′,3′ - diméthylbutyrolactone 2′ - (R) oxy/ 3 - oxabicyclo /3.1.0/ hexan 2 - one F = 134 °C et, enfin, 1,654 g de (1R, 5S) 6,6 - diméthyl 4(R)/ 3′,3′ - diméthylbutyrolactone 2′ - (R) oxy/ 3 - oxabicyclo /3.1.0/ hexan - 2 - one F = 120 °C (composé B).
Spectres R.M.N. ($CDCl_3$)
Composé A
Pic à 4,0 p.p.m. caractéristique des hydrogènes en position 4′ de la pantolactone.
Pic à 4,2 p.p.m. caractéristique de l'hydrogène en position 2′ de la pantolactone.
Pic à ≃ 5,7 p.p.m. caractéristique de l'hydrogène en position 4 du bicyclo /3.1.0/ hexanone.
Pics à 2-2,1 et 2,22-2,31 p.p.m. caractéristiques des hydrogènes en positions 1 et 3 du cyclopropyle.
Pics à 1,1-1,22 p.p.m. caractéristiques des hydrogènes des méthyles.
Composé B
Pic à 4,0 p.p.m. caractéristique des hydrogènes en position 4′ de la pantolactone.
Pic à 4,23 p.p.m. caractéristique de l'hydrogène en position 2′ de la pantolactone.
Pic à 5,47 p.p.m. caractéristique de l'hydrogène en position 4 du bicyclo /3.1.0/ hexanone.
Pics à 2,06-2,15 et 2,36-2,45 p.p.m. caractéristiques des hydrogènes en 1 et 3 du cyclopropyle.
Pics à 1,12-1,2-1,24 p.p.m. caractéristiques des hydrogènes des méthyles.

Exemple 15 : (3S, 3aR, 4S, 7R, 7aS) 3-/(1S) 2 - méthyl 4 - oxo 3 - (2 - propen 1 -yl) cyclopent 2 - ényloxy/tétrahydro 4,7 méthano isobenzofuran - 1 - one et,
(3R, 3aS, 4R, 7R, 7aR) 3-/(1S) 2 - méthyl 4 - oxo 3 - (2 - propen 1 - yl) cyclopent 2 - ényloxy/tétrahydro 4,7 méthano isobenzofuran 1 - one, sous forme de mélange racémique.

On chauffe au reflux, pendant 3 heures, 66 g de 3 - hydroxy tétrahydro 4,7 - méthano isobenzofuran 1 - one racémique préparés comme à l'exemple 12, 60 g de 1(S) hydroxy 2 - méthyl 3 - (2 - propen 1 - yl) cyclopent 2 - ène 4 - one, 600 $cm^3$ de benzène et 300 mg d'acide paratoluène sulfonique. On neutralise avec 4 $cm^3$ de triéthylamine puis concentre à sec. On reprend à l'éther éthylique et essore 23 g d'un produit cristallisé F = 148 °C correspondant au (3R, 3aR, 4S, 7R, 7aS) 3-/(1S) 2 - méthyl 4 - oxo 3 - (2 - propen 1 - yl) cyclopent 2 - ényloxy/tétrahydro 4,7 - méthano isobenzofuran 1 - one (composé A obtenu à l'exemple 12). Les liqueurs mères sont chromatographiées sur silice et éluées avec un mélange chloroforme-acétone (100-5).

On isole successivement 28,8 g de (3S, 3aS, 4R, 7S, 7aR) 3-/(1S) 2 - méthyl 4 - oxo 3 - (2 - propen 1 - yl) cyclopent 2 - ényloxy/tétrahydro 4,7 - méthano isobenzofuran 1 - one (composé B obtenu à l'exemple 12) puis 26 g de composé A et enfin 16 g d'un mélange de diastéréoisomères endo recherché.
Spectre I.R. ($CHCl_3$)

| | | |
|---|---|---|
| γ lactone | = absorption à | 1764 $cm^{-1}$ |
| alléthrolone | = absorptions à | 1708 $cm^{-1}$ |
| | = | 1655 $cm^{-1}$ |
| | = | 1639 $cm^{-1}$ |

région squelette très différente des produits A et B
Spectre R.M.N. ($CDCl_3$)
Pic à 6,17 p.p.m. caractéristique des hydrogènes éthyléniques en positions 5 et 6.
Pics de 2,92 à 3,42 p.p.m. caractéristiques des hydrogènes en position 3a, 4,7 et 7a.
Pics à 1,33-1,48 et 1,55-1,7 p.p.m. caractéristiques des hydrogènes en position 8.
Pics de 5,58 à 5,75 p.p.m. caractéristiques de l'hydrogène en position 3.
Pics de 4,5 à 4,83 p.p.m. caractéristiques de l'hydrogène en 1′ dans l'alléthrolone.

Pic à 2,07 p.p.m. caractéristique des hydrogènes du méthyle en 2' de l'alléthrolone.
Pics de 5,52 à 6,17 p.p.m. caractéristiques de l'hydrogène en 2 du propényle.
Pics de 4,83 à 5,17 p.p.m. caractéristiques des hydrogènes en 3 du propényle.
Composés différents des produits A et B.

Exemple 16 : (1R, 5S) 6,6 - diméthyl 4-(R)/ (R) éthynyl - (3' - phénoxy phényl) méthoxy/ 3 - oxabicyclo /3.1.0/ hexan 2 - one (isomère R).

Pendant 1 heure, sous agitation, on chauffe au reflux en éliminant l'eau formée par azéotropie, 20 g de lactone de l'acide cis 2,2 - diméthyl 3S - (dihydroxy méthyl) cyclopropane 1R - carboxylique, 200 $cm^3$ de benzène, 30 g d'alcool éthynyl (3' - phénoxyphényl) méthylique racémique et 200 mg d'acide paratoluène sulfonique. On laisse refroidir à 20 °C puis neutralise par addition de triéthylamine. On concentre à sec sous pression réduite et obtient 51 g d'une huile brune. Par chromatographie sous pression puis élution par mélange benzène-acétate (95-5) on isole 10,7 g d'isomère R sous forme d'une huile.

Caractéristiques de l'isomère R :
Spectre R.M.N. ($CDCl_3$)
Pics à 1,16-1,25 p.p.m. caractéristiques des hydrogènes des méthyles géminés.
Pic à 5,65 p.p.m. caractéristique de l'hydrogène en 4.
Pics à 2,68-2,7 p.p.m. caractéristiques de l'hydrogène de l'éthynyle.
Pics à 5,50-5,52 p.p.m. caractéristiques de l'hydrogène porté par le même carbone que l'éthynyle.
Pics de 6,83 à 7,50 p.p.m. caractéristiques des hydrogènes des noyaux aromatiques.
Pics à 1,95-2,03 et 2,1-2,18 p.p.m. caractéristiques des hydrogènes du cyclopropyle.

Exemple 17 : (1R, 5S) 6,6 - diméthyl 4-(R)/(S) éthynyl - (3' - phénoxy phényl) méthoxy/ 3 - oxabicyclo /3.1.0/ hexan 2 - one (isomère S).

En poursuivant la chromatographie sous pression, décrite à l'exemple 16, on isole 10,4 g d'isomère S sous forme d'une huile, puis 8,4 g d'un mélange d'isomère R et S.

Caractéristiques de l'isomère S :
Spectre R.M.N. ($CDCl_3$)
Pic à 1,11 p.p.m. caractéristique des hydrogènes des méthyles géminés.
Pic à 5,05 p.p.m. caractéristique de l'hydrogène en 4.
Pics à 2,65-2,68 p.p.m. caractéristiques de l'hydrogène de l'éthynyle.
Pics à 5,41-5,45 p.p.m. caractéristiques de l'hydrogène porté par le même carbone que l'éthynyle.
Pics à 2,05 p.p.m. caractéristique des hydrogènes du cyclopropyle.

Exemple 18 : (1R, 5S) 6,6 - diméthyl 4(R)-/3' - méthyl 2'(R) butoxy / 3 - oxabicyclo /3.1.0/ hexan 2 - one.

Pendant deux heures on chauffe au reflux, sous agitation 28 g de lactone de l'acide cis 2,2 - diméthyl 3S - (dihydroxyméthyl) cyclopropane - 1R - carboxylique, 100 $cm^3$ de benzène, 100 mg d'acide paratoluène sulfonique et 25 $cm^3$ de 3 - méthyl - 2 - butanol racémique. L'eau formée au cours de la réaction est éliminée par azéotropie. On laisse refroidir à 20 °C, puis neutralise avec la triéthylamine. On concentre à sec sous pression réduite et obtient 44 g d'huile. Par chromatographie de cette huile sur silice on obtient 33 g d'un mélange des deux diastéréoisomères. On chromatographie sous pression, sur silice, 360 mg de celui-ci, élue avec du chlorure de méthylène à 2 % d'acétonitrile et recueille 160 mg du diastéréoisomère R · F ≃ 19-21 °C.
$/\alpha/^{20}_D = -143° \pm 3{,}5°$ (C = 0,5 % benzène)
Spectre R.M.N. ($CDCl_3$)
Pics à 1,15-1,17 p.p.m. caractéristiques des hydrogènes des méthylènes géminés.
Pic à 5,25 p.p.m. caractéristique de l'hydrogène en 4.
Pics à 1,07-1,17 p.p.m. caractéristiques des hydrogènes du méthyle en 1' du propyloxy.
Pics de 3,45 à 3,87 p.p.m. caractéristiques de l'hydrogène en 1' du propyloxy.
Pics de 0,83 à 0,93 p.p.m. caractéristiques des hydrogènes des méthyles en 2', 3' du propyloxy.

Exemple 19 : (1R, 5S) 6,6 - diméthyl 4(R)/3' - méthyl 2'(S) butyoxy/ 3 - oxabicyclo /3.1.0/ hexan - 2 - one.

Après avoir recueilli l'isomère A à l'exemple 18, on poursuit l'élution pour obtenir 57 mg d'isomère S · F ≃ 35 °C.
$/\alpha/^{20}_D = -121° \pm 3{,}5°$ (C = 0,5 % benzène)
Spectre R.M.N. ($CDCl_3$)
Pics à 1,17-1,19 p.p.m. caractéristiques des hydrogènes des méthyles géminés.
Pic à 5,2 p.p.m. caractéristique de l'hydrogène en 4.
Pics de 3,38 à 3,46 p.p.m. caractéristiques de l'hydrogène en 1' du propyloxy.
Pics à 0,83-0,95 p.p.m. caractéristiques des hydrogènes des méthyles en 2' et 3' du propyloxy.

Pics à 1,15-1,25 p.p.m. caractéristiques des hydrogènes du méthyle en 1'.

Exemple 20 : (3S, 3aS, 4R, 7S, 7aR) 3-/1(R) 3' - (phénoxyphényl) α - méthyl - méthoxy tétrahydro 4,7 - méthano isobenzofuran 1 - one.

On chauffe au reflux pendant 1 heure et demie 300 mg de (3S, 3aS, 4R, 7S, 7aR) 3 - hydroxy tétrahydro 4,7 - méthano isobenzofuran 1 - one obtenu ci-après à l'exemple 25, 30 $cm^3$ de benzène, 400 mg de 1(R) (3' - phénoxyphényl) α - méthyl méthanol et 30 mg d'acide paratoluène sulfonique. Après refroidissement à 20 °C, on neutralise avec de la triéthylamine et concentre à sec sous pression réduite. L'huile obtenue est chromatographiée sur silice puis éluée par un mélange benzène-acétate d'éthyle (9-1). On obtient 400 mg de produit F = 146 °C.

Spectre R.M.N. ($CDCl_3$)

Pics à 1,3-1,65 p.p.m. caractéristiques des hydrogènes du $CH_2$ en 8.
Pics à 5,83-6,25 p.p.m. caractéristiques des hydrogènes éthyléniques.
Pic à 4,7 p.p.m. caractéristique de l'hydrogène en position 3.
Pics à 1,37-1,48 p.p.m. caractéristiques des hydrogènes du méthyle en α.
Pics à 4,58-4, 7-4, 82-4,93 p.p.m. caractéristiques de l'hydrogène en 1' de la copule alcoolique.
Pics à 6,83-7,5 p.p.m. caractéristiques des hydrogènes des noyaux aromatiques.

Dichroïsme circulaire (dioxane)

max. 235 nm $\Delta\ \varepsilon = +1,9$
infl. 257 nm $\Delta\ \varepsilon = +0,58$
max. 263 nm $\Delta\ \varepsilon = +0,77$
max. 276 nm $\Delta\ \varepsilon = -1,67$
max. 281 nm $\Delta\ \varepsilon = -2,00$

Structure R au niveau 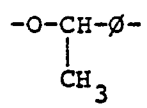

Exemple 21 : (3S, 3aS, 4R, 7S, 7aR) 3/1(S) (3' - phénoxyphényl) α - méthyl méthoxy/tétrahydro 4,7 méthano isobenzofuran 1 - one.

On chauffe au reflux, pendant 1 h 30, sous agitation, 300 mg de (3S, 3aS, 4R, 7S, 7aR) 3 - hydroxy tétrahydro 4,7 méthano isobenzofuran 1 - one obtenu ci-après à l'exemple 25, 15 $cm^3$ de benzène et 400 mg de 1(S) (3' - phénoxyl) α - méthyl méthanol et 30 mg d'acide paratoluène sulfonique. Après refroidissement, on concentre à sec sous pression réduite et obtient 900 mg d'une huile épaisse. On chromatographie celle-ci en éluant par un mélange benzène-acétate d'éthyle (95-5). On recueille 400 mg de produit qui cristallise F = 78-79 °C.

Spectre I.R. ($CHCl_3$)
Absorption C = O α-lactone à 1769 $cm^{-1}$,
Absorption des noyaux aromatiques à 1587-1490 $cm^{-1}$,
Absorption C-O-C à 1250 $cm^{-1}$,

Absorption 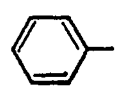 694 $cm^{-1}$.

Dichroïsme circulaire (dioxane)

max. 281 nm $\Delta\ \varepsilon$ $+0,8$
max. 275 nm $\Delta\ \varepsilon$ $+0,6$
max. $\simeq$ 225 nm $\Delta\ \varepsilon \sim -2,5$ à $-3$

compatible avec la configuration S pour la partie alcool.

Spectre R.M.N. ($CDCl_3$)

Pic à 6,2 p.p.m. caractéristique des hydrogènes éthyléniques.
Pics à 1,33-1,75 p.p.m. caractéristiques des hydrogènes du $CH_2$ en 8.
Pic à 5,07 p.p.m. caractéristique de l'hydrogène en position 3.
Pics à 1,4-1,5 p.p.m. caractéristiques des hydrogènes de α-méthyle.
Pics à 4,6-4, 7-4,8 p.p.m. caractéristiques de l'hydrogène porté par le même carbone que α-méthyle.
Pics à 6,7-7,5 p.p.m. caractéristiques des hydrogènes des noyaux aromatiques.
Pics de 2,67 à 3,5 p.p.m. caractéristiques des autres protons.

Exemple 22 : 1(S) hydroxy 2 - méthyl 3 - (2 - propen 1 - yl) cyclopent 2 - ène 4 - one (ou S alléthrolone).

Dans 20 $cm^3$ de dioxane on introduit 2,7 g de (3R, 3aR, 4S, 7R, 7aS) 3-/(1S) 2 - méthyl 4 - oxo 3 - (2 - propen 1 - yl) cyclopent 2 - ényloxy/tétrahydro 4,7 - méthano isobenzofuran 1 - one obtenu à l'exemple 12, 30 $cm^3$ d'eau et 0,25 g d'acide paratoluène sulfonique monohydraté, chauffe au reflux pendant 24 heures. Après élimination du solvant sous pression réduite, on neutralise avec de la triéthylamine jusqu'à pH 7, puis concentre à sec sous pression réduite. On obtient 2,8 g de produit brut. Celui-ci est chromatographié sur silice et élué par un mélange benzène-acétate d'éthyle (1-1) à 1 pour 1 000 de triéthylamine. On recueille 1 g d'alléthrolone de configuration S, 0,4 g de lactone et 0,7 g d'un mélange lactone-alléthrolone.

$/\alpha/^{20}_D = +6{,}5° \pm 1°$ (C = 1,5 % benzène).

Dichroïsme circulaire (dioxane)

max. 228 nm $\Delta\varepsilon$ : − 15,7
max. 318-319 nm $\Delta\varepsilon$ : + 2,46
max. 330 nm $\Delta\varepsilon$ : + 2,26

Exemple 23 : 1(R) - hydroxy 2 - méthyl 3 - (2 - propen 1 - yl) cyclopent 2 - ène 4 - one (ou R alléthrolone).

En opérant de la même manière qu'à l'exemple précédent, mais à partir du (3R, 3aR, 4S, 7R, 7aS) 3-/(1R) 2 - méthyl 4 - oxo 3 - (2 - propen 1 - yl) cyclopent 2 - ényloxy/tétrahydro 4,7 - méthano isobenzofuran 1 - one obtenu à l'exemple 13, on obtient de la R alléthrolone avec le même rendement.

$/\alpha/^{20}_D = -6{,}5° \pm 1°$ (C = 0,8 benzène).

Dichroïsme circulaire (dioxane)

max. 228 nm $\Delta\varepsilon$ + 17,3
max. 318 nm $\Delta\varepsilon$ − 2,92
max. 330 nm $\Delta\varepsilon$ − 2,68

Exemple 24 : (3R, 3aR, 4S, 7R, 7aS) 3 - hydroxy tétrahydro 4,7 - méthano isobenzofuran 1 - one.

Dans 60 $cm^3$ de dioxane on introduit 12,8 g de (3R, 3aR, 4S, 7R, 7aS) 3-/(1S)2 - méthyl 4 - oxo 3 - (2 - propen 1 - yl) cyclopent 2 - ényloxy/tétrahydro 4,7 - méthano isobenzofuran 1 - one obtenu à l'exemple 12, 120 $cm^3$ d'eau et 1,2 g d'acide paratoluène sulfonique monohydraté. On chauffe au reflux pendant 2 heures. On neutralise à pH 7 avec de la triéthylamine et concentre à sec sous pression réduite. Le produit brut est chromatographié sur silice en éluant par un mélange benzène-acétate d'éthyle (1-1) contenant 1 pour mille de triéthylamine. On obtient 5,3 g du produit attendu sous forme de cristaux blancs fondant à 120 °C.

Analyse : $C_9H_{10}O_3$ PM : 166,17

| | C % | H % |
|---|---|---|
| Calculé | 65,05 | 6,07 |
| Trouvé | 65,0 | 6,1 |

Dichroïsme circulaire (dioxane)

max. 218 nm $\Delta\varepsilon$ = + 2,57
max. 292 nm $\Delta\varepsilon$ = − 0,015
max. 333 nm $\Delta\varepsilon$ = + 0,011
max. 345 nm $\Delta\varepsilon$ = + 0,006

$/\alpha/^{20}_D = +49{,}5°$ (C = 1 %, chloroforme)

Spectre I.R. ($CHCl_3$)

Absorption à 3580 $cm^{-1}$ caractéristique de l'hydroxyle,

Absorption à 1769-1740 $cm^{-1}$ caractéristique du carbonyle.

Spectre R.M.N.

Pic à 6,2 p.p.m. caractéristique des hydrogènes éthyléniques.
Pics à 5,22-5,23 p.p.m. caractéristiques de l'hydrogène en position 3.
Pic à 4,75 p.p.m. caractéristique de l'hydrogène de l'hydroxyle.
Pics à 1,33-1,73 p.p.m. caractéristiques des hydrogènes du $CH_2$ en 8.
Pics à 2,75-3,52 p.p.m. caractéristiques des autres protons.

Exemple 25 : (3S, 3aS, 4R, 7S, 7aR) 3 - hydroxy tétrahydro 4,7 - méthano isobenzofuran 1 - one.

Dans 20 $cm^3$ de dioxane on introduit 1,9 g de (3S, 3aS, 4R, 7S, 7aR) 3/(1R) 2 - méthyl 4 - oxo 3 - (2 -

propen 1 - yl) cyclopent 2 - ényl-oxy/tétrahydro 4,7 - méthano isobenzofuran 1 - one obtenu à l'exemple 13, 20 $cm^3$ d'eau et 0,2 g d'acide paratoluène sulfonique monohydraté. On chauffe au reflux pendant 6 heures. On neutralise jusqu'à pH 7 avec de la triéthylamine et concentre à sec sous pression réduite. Le résidu est chromatographié sur silice, élué par un mélange benzène-acétate d'éthyle (1-1) à 1 pour mille de triéthylamine. On obtient 650 mg de produit fondant à 120 °C présentant les caractéristiques suivantes :

$/\alpha/^{20}_D = -47,5° \pm 2,5°$ (C = 1 %, chloroforme).

Spectre R.M.N. ($CDCl_3$)

Pic à 6,23 p.p.m. caractéristique des hydrogènes éthyléniques.
Pic à 5,25 p.p.m. caractéristique de l'hydrogène en position 3.
Pic à 4,9 p.p.m. caractéristique de l'hydrogène du OH.
Pics à 1,35-1,5 et 1,6-1,75 p.p.m. caractéristiques des hydrogènes du $CH_2$ en position 8.
Pics de 2,83 à 3,58 p.p.m. caractéristiques des autres protons.

Exemple 26 : (3S, 3aR, 4S, 7R, 7aS) 3 - hydroxy tétrahydro 4,7 - méthano isobenzofuran 1 - one et (3R, 3aS, 4R, 7S, 7aR) 3 - hydroxy tétrahydro 4,7 - méthano isobenzofuran 1 - one, sous forme d'un mélange racémique.

On chauffe au reflux, pendant 2 heures, 15 g du mélange obtenu à l'exemple 15 ((3S, 3aR, 4S, 7R, 7aS) 3-/(1S) 2 - méthyl 4 - oxo 3 - (2 - propen 1 - yl) cyclopent 2 - ényloxy/tétrahydro 4,7 - méthano isobenzofuran 1 - one et (3R, 3aS, 4R, 7S, 7aR) 3-/(1S) 2 - méthyl 4 - oxo 3 - (2 - propen 1 - yl) cyclopent 2 - ényloxy/tétrahydro 4,7 - méthano isobenzofuran 1 - one), 75 $cm^3$ de dioxane, 150 $cm^3$ d'eau et 1,5 g d'acide paratoluène sulfonique. On refroidit, ajuste le pH à 7-8 avec de la triéthylamine et concentre à sec sous pression réduite. L'huile obtenue est chromatographiée sous pression sur silice, éluée par un mélange benzène-acétate d'éthyle (1-1) à 1 pour 1 000 de triéthylamine. On obtient 4,85 g de produit F = 103 °C.

Spectre R.M.N. ($CDCl_3$)

Pic 6,2 p.p.m. caractéristique des hydrogènes éthyléniques.
Pic 5,22 p.p.m. caractéristique de l'hydrogène en position 3.
Pics à 1,33-1,48 et 1,55-1,7 p.p.m. caractéristiques des hydrogènes du $CH_2$ en 8.
Pic à 4,67 p.p.m. caractéristique de l'hydrogène du OH.
Pics de 2,75 à 3,5 p.p.m. caractéristiques des autres protons.
Pas de dichroïsme à 218 nm pour le chromophore lactone, donc produit racémique.

Exemple 27 : (S) - 1 - (3 - phénoxy phényl) prop - 2 - yn 1 - ol.

Dans un mélange de 80 $cm^3$ de dioxane et de 80 $cm^3$ d'eau, on introduit 10,4 g de (1R, 5S) 6,6 - diméthyl 4(R)/(S)- éthynyl (3' - phénoxy phényl - méthoxy/3 - oxabicyclo /3.1.0/ hexan - 2 - one obtenus à l'exemple 17, 1 g d'acide paratoluène sulfonique, porte le mélange réactionnel au reflux, maintient le reflux pendant 2 heures, concentre presque à sec, par distillation sous pression réduite, ajoute de l'eau, agite, extrait à l'éther isopropylique, effectue les traitements habituels, concentre à sec par distillation sous pression réduite, chromatographie le résidu (6 g) sur gel de silice en éluant par un mélange de benzène et d'acétate d'éthyle (7-3) et obtient 5,1 g de (S) 1 - (3 - phénoxy phényl) prop - 2 - yn 1 - ol.

$/\alpha/^{20}_D = +10,5°$ (C = 0,63 %, benzène).

Spectre R.M.N. (deuterochloroforme)

Pic à 2,30 p.p.m. caractéristique de l'hydrogène de l'hydroxyle.
Pics à 2,61-2,66 p.p.m. caractéristiques de l'hydrogène éthylénique.
Pics 5,44-5,50 p.p.m. caractéristiques de l'hydrogène porté par le même carbone que le groupement éthynyl.
Pics à 6,91-7,0 p.p.m. caractéristiques des hydrogènes des noyaux aromatiques.

Dichroïsme circulaire (dioxane)

$\Delta\varepsilon = +1,9$ à 215 nm (max.)
$\Delta\varepsilon = +0,02$ à 272 nm (max.)
$\Delta\varepsilon = -0,01$ à 276 nm (max.)
$\Delta\varepsilon = +0,04$ à 278 nm (max.)
$\Delta\varepsilon = -0,03$ à 283 nm (max.)

Exemple 28 : (R) 1 - (3 - phénoxy phényl) prop - 2 - yn - 1 - ol.

En opérant de façon analogue à celle de l'exemple 27 mais au départ de 10,6 g de (1R, 5S) 6,6 - diméthyl 4(R)/(R) - éthynyl (3' - phénoxy phényl) méthoxy/3 - oxabicyclo /3.1.0/ hexan - 2 - one obtenus à l'exemple 16, on obtient 5,6 g de (R) 1 - (3 - phénoxy phényl) prop - 2 - yn - 1 - ol.

$/\alpha/^{20}_D = -16°$ (C = 1 %, benzène).

Spectre R.M.N. (deuterochloroforme)

Pics à 2,58-2,62 p.p.m. caractéristiques de l'hydrogène acétylènique.
Pic à 2,66 p.p.m. caractéristique de l'hydrogène de l'hydroxyle.
Pics à 5,36-5,40 p.p.m. caractéristiques de l'hydrogène porté par le même carbone que le groupement éthynyle.
Pics à 6,83-7,50 p.p.m. caractéristique des hydrogènes des noyaux aromatiques.

Exemple 29 : 3 - méthyl 2 - (S) butanol.

On chauffe à 50 °C, pendant 16 heures, 16,9 g de (1R, 5S) 6,6 - diméthyl 4(R)/3 - méthyl 2(S) - butoxy/3 - oxabicyclo /3.1.0/ hexan - 2 - one, obtenus à l'exemple 19, 70 $cm^3$ d'acide chlorhydrique 2N et 8,5 $cm^3$ d'acétone. On laisse refroidir à 20 °C puis extrait à l'éther. Les extraits organiques réunis sont lavés avec de l'ammoniaque ~2N puis à l'eau jusqu'à neutralité, sèchés, puis concentrés à sec sous pression réduite. On obtient une huile que l'on purifie par distillation soit 1,7 g de produit Eb/760 = 108 °C.
$/\alpha/^{20}_D = + 3° \pm 1°$ (C = 1 % éthanol).
Spectre R.M.N. ($CDCl_3$)
Pic à 1,5 p.p.m. caractéristique de l'hydrogène de l'hydroxyle.
Pics à 1,08-1,2 p.p.m. caractéristiques des hydrogènes du méthyle en 1.
Pic à 3,58 p.p.m. caractéristique de l'hydrogène en 2.
Pic à 1,58 p.p.m. caractéristique de l'hydrogène en 3.
Pics à 0,85-0,95 p.p.m. caractéristiques des hydrogènes des méthyles en 3 et 4.

Exemple 30 : 3 - méthyl 2 - (R) butanol.

On chauffe à 50 °C sous agitation pendant 16 heures, 16 g de (1R, 5S) 6,6 - diméthyl 4(R)/3' - méthyl 2'(R) butoxy/3 - oxabicyclo /3.1.0/ hexan - 2 - one obtenus à l'exemple 18, 70 $cm^3$ d'acide chlorhydrique 2N et 10 $cm^3$ d'acétone. On laisse refroidir à 20 °C et on extrait à l'éther. Les extraits organiques réunis sont lavés à l'ammoniaque ~2N, à l'eau jusqu'à neutralité, sèchés et concentrés à sec sous pression réduite. On obtient 7 g de produit brut que l'on distille pour obtenir 1,5 g d'huile Eb/760 = 110 °C.
$/\alpha/^{20}_D = - 4,5° \pm 1°$ (C = 1,3 % éthanol)
Spectre R.M.N. ($CDCl_3$)
Pics à 1,1-1,2 p.p.m. caractéristiques des hydrogènes du méthyle en 1.
Pic à 1,67 p.p.m. caractéristique de l'hydrogène du OH.
Pic à 3,58 p.p.m. caractéristique de l'hydrogène en 2.
Pic à 1,67 p.p.m. caractéristique de l'hydrogène en 3.
Pics à 0,85-0,97 p.p.m. caractéristiques des hydrogènes des méthyles en 3 et 4.

Exemple 31 : 2 - (S) - hydroxy 3,3 - diméthyl butyrolactone ou (S) pantolactone.

On chauffe au reflux pendant 2 heures et sous agitation 20 g de (1R, 5S) 6,6 - diméthyl 4 - (R) -/3',3' - diméthylbutyrolactone 2' - (S) oxy/3 - oxa bicyclo /3.1.0/ hexan 2 - one, obtenus à l'exemple 14, 100 $cm^3$ d'eau, 100 $cm^3$ de dioxane et 1 g d'acide paratoluène sulfonique monohydraté. On refroidit à température ambiante, ajoute 100 $cm^3$ d'eau et concentre par distillation sous pression réduite jusqu'à environ 50 $cm^3$. On sature de chlorure de sodium et extrait au chlorure de méthylène. Les phases organiques séchées sont concentrées à 40 °C sous pression réduite.

On récupère un produit blanc qui est un mélange de (1R, 5S) 6,6 - diméthyl 4(R) - hydroxy 3 - oxabicyclo /3.1.0/ hexan 2 - one (ou produit A) et du produit attendu. On le dissout au reflux dans 40 $cm^3$ d'eau. Après refroidissement à température ambiante on amorce la cristallisation du produit A avec quelques cristaux et agite pendant 16 heures. On essore, lave à l'eau, concentre le filtrat à sec, traite comme précédemment en refroidissant à 0° + 5 °C pendant 2 heures pour récupérer encore du produit °C.

Le dernier filtrat concentré à sec est chromatrographié sur silice et élué par un mélange toluène-acétate d'éthyle (6-4).

On récupère 7 g de cristaux blancs contenant encore du produit A. On en reprend 6 g que l'on dissout dans 50 $cm^3$ d'eau et 2 $cm^3$ d'éthanol, ajoute 25 $cm^3$ de solution de bisulfite de sodium et agite énergiquement pendant 2 heures à température ambiante. On sature de chlorure de sodium et extrait à l'acétate d'éthyle. Les phases organiques séchées sont concentrées à sec sous pression réduite à 40 °C. Les 4,5 g de produit obtenu sont sublimés à 90 °C sous 0,1 mm de mercure. On obtient 2,7 g du produit attendu.
F ≃ 90 °C.
$/\alpha/^{20}_D = + 47,5° \pm 1°$ (C = 2 % - eau)
Spectre R.M.N. ($CDCl_3$)
Pics à 1,08-1,22 p.p.m. caractéristiques des hydrogènes des méthyles géminés.
Pic à ~ 3,77 p.p.m. caractéristique de l'hydrogène de l'hydroxyle.
Pic à 3,98 p.p.m. caractéristique des hydrogènes du méthylène en 4 du cyclopentyle.
Pic à 4,18 p.p.m. caractéristique de l'hydrogène en position 2 du cyclopentyle.

Exemple 32 : 2 - (R) hydroxy 3,3 - diméthyl butyrolactone ou (R) pantolactone.

On chauffe au reflux pendant 2 heures et sous agitation 9 g de (1R, 5S) 6,6 - diméthyl 4(R)/3',3' - diméthyl butyrolactone 2' (R) oxy/ 3 - oxabicyclo /3.1.0/ hexan 2 - one obtenu à l'exemple 14, 90 $cm^3$ de méthanol et 85 mg d'acide paratoluène sulfonique monohydraté. Le mélange réactionnel est concentré à sec sous pression réduite puis chromatographié sur silice en éluant avec un mélange toluène-acétate d'éthyle (6-4). On recueille 1,3 g de cristaux jaunes qui sont sublimés à 90°-100 °C sous 0,1 mm de mercure. On obtient 1 g de cristaux blancs hygroscopiques du produit attendu. F = 89 °C.
$/\alpha/^{20}_D = -50° \pm 1°$ (C = 1,96 %, eau).

Exemple 33 : (3R) (3aR) (4S) (7R) (7aS) 3-/(1'R) (2'S) (5'R) 2' - isopropyl 5' - méthylcyclohexanoxy/ - tétrahydro 4,7 - méthano isobenzofuran 1 - one.

Pendant 1 h 1/2 on chauffe au reflux sous agitation, 1,66 g de (3R) (3aR) (4S) (7R) (7aS) 3 - hydroxy tétrahydro 4,7 - méthano isobenzofuran 1 - one, 2,34 g de 1 - menthol, 50 mg d'acide paratoluène sulfonique et 42 $cm^3$ de benzène. Après retour à température ambiante, le milieu réactionnel est concentré à sec sous pression réduite. On obtient 4,41 g d'huile qui est chromatographiée sur silice en éluant par un mélange benzène-acétate d'éthyle (95-5). On obtient 2,76 g de produit attendu (F = 54 °C et 92 mg du diastéréoisomère S (F = 100 °C).
Spectre I.R. ($CHCl_3$)

C = O $\gamma$-lactone absorption 1769 $cm^{-1}$ max.
1760 $cm^{-1}$ épaul.

méthyles géminés 1390 $cm^{-1}$
- C - O - C (éther) 1117 $cm^{-1}$
Spectre R.M.N. ($CDCl_3$)
Pics à 0,68-0, 8-0,92 p.p.m. caractéristiques des hydrogènes des méthyles de l'isopropyle.
Pics à 0,8 8-0,97 p.p.m. caractéristiques des hydrogènes du méthyle du menthol.
Pics à 2,67-3,67 p.p.m. caractéristiques de l'hydrogène en 1' du menthol et des hydrogènes en 3a, 4, 7 et 7a de la lactone.
Pics à 5,03-5,06 p.p.m. caractéristiques de l'hydrogène en 3 de la lactone.
Pic à 6,22 p.p.m. caractéristique des hydrogènes éthyléniques.
Dichroïsme circulaire (dioxane)
Max. à 217 nm $\Delta\varepsilon = +2,3$
$/\alpha/^{20}_D = -120,5° \pm 2°$ (C = 1 % benzène)

Exemple 34 : (3R), (3aR), (4S), (7R), (7aS) 3-/(1'R), (2'S) (5'R), 2' - isopropyl 5' - méthylcyclohexanoxy/tétrahydro 4,7 - méthano isobenzofuran 1 - one (produit R) et
(3R) (3aR) (4S) (7R) (7aS) 3-/(1'S) (2'R) (5'S) 2' - isopropyl 5' - méthylcyclohexanoxy/tétrahydro 4,7 - méthano isobenzofuran 1 - one (produit S).

On chauffe au reflux pendant 2 heures 6,65 g de (3R) (3aR) (4S) (7R) (7aS) 3 - hydroxy tétrahydro 4,7 - méthano isobenzofuran 1 - one, 9,37 g de menthol racémique, 200 mg d'acide paratoluène sulfonique monohydraté et 170 $cm^3$ de benzène. Le mélange réactionnel est concentré à sec sous pression réduite à 40 °C et l'on obtient 16,17 g d'huile brune.

Pour séparer les constituants de ce produit brut on effectue une chromatographie sous pression et une chromatographie à pression atmosphérique. On élue avec un mélange benzène-acétate d'éthyle (95-5) pour la première et (98-2) pour la seconde. On recueille 3,58 g de produit blanc F = 53 °C correspondant au produit R, 4,25 g d'une huile épaisse correspondant au produit S et 0,57 g d'une cire blanche constituée d'un mélange des produits R et S.

Diastéréoisomère R :
Spectre R.M.N. ($CDCl_3$)
Pics à 0,7-0,82-0,93 p.p.m. caractéristiques des hydrogènes des méthyles de l'isopropyle.
Pics à 0,88-0,97 p.p.m. caractéristiques des hydrogènes du méthyle.
Pics à 5,05-5,07 p.p.m. caractéristiques de l'hydrogène en 3 de la lactone.
Pic à 6,23 p.p.m. caractéristique des hydrogènes éthyléniques.
Pics à 2,67-3,67 p.p.m. caractéristiques de l'hydrogène en 1 du menthol et des hydrogènes en 4, 3a, 7 et 7a de la lactone.
$/\alpha/^{20}_D = -113,5° \pm 3°$ (C = 0,66 % benzène).

Diastéréoisomères S :
Spectre R.M.N. ($CDCl_3$)
Pics à 0,75-0,87-0,98 p.p.m. caractéristiques des hydrogènes des méthyles de l'isopropyle.
Pics à 0,92-0,87 p.p.m. caractéristiques des hydrogènes du méthyle.
Pics à 4,92-4,95 p.p.m. caractéristiques de l'hydrogène en 3 de la lactone.
Pic à 6,23 p.p.m. caractéristiques des hydrogènes éthyléniques.

$/\alpha/^{20}_D = -74° \pm 3°$ (C = 0,3 % benzène).

Exemple 35 : (1R) (2S) (5R) 2 - isopropyl 5 - méthyl cyclohexanol

On chauffe au reflux pendant 1 heure sous agitation 1,78 g de (3R) (3aR) (4S) (7R) (7aS) 3-/(1'R) (2'S) (5'R) 2' - isopropyl 5' - méthylcyclohexanoxy/ tétrahydro 4,7 - méthano isobenzofuran 1 - one, 25 $cm^3$ d'eau, 300 mg d'acide paratoluène sulfonique monohydraté et 40 $cm^3$ de dioxane. On ajoute 100 $cm^3$ d'eau et distille à 40 °C sous pression réduite le maximum de dioxane. On extrait à l'éther éthylique, lave à l'eau, sèche et concentre à sec sous pression réduite pour obtenir 1,12 g d'huile. Le produit brut est chromatographié sur silice, élué par un mélange benzène-acétate d'éthyle (95-5). On isole 750 mg de produit attendu $F < 50$ °C.

Spectre I.R. ($CHCl_3$)

Méthyles géminés : absorption à 1370 $cm^{-1}$

fonction alcool : absorption à 3595 $cm^{-1}$

3610 $cm^{-1}$

$/\alpha/^{20}_D = -49° \pm 2{,}5°$ (C = 0,7 % éthanol)

Exemple 36 : (1R, 5S) 6,6 - diméthyl (4R) -/(1'R) (2'S) (5'R) 2' - isopropyl 5' - méthylcyclohexanoxy/ 3 - oxabicyclo /3.1.0/ hexan 2 - one (diastéréoisomère R) et
(1R, 5S) 6,6 - diméthyl 4R-/(1'S) (2'R) (5'S) 2' - isopropyl 5' - méthyl cyclohexanoxy/ 3 - oxabicyclo /3.1.0/ hexan 2 - one (diastéréoisomère S).

On chauffe au reflux, pendant 1 h 30, sous agitation, 7,3 g de (1R, 5S) 6,6 - diméthyl 4(R) hydroxy 3 - oxabicyclo /3.1.0/ hexan 2 - one, 100 $cm^3$ de benzène, 7,8 g de menthol racémique et 100 mg d'acide paratoluène sulfonique. Le mélange réactionnel est refroidi à température ambiante, neutralisé par addition de 2 $cm^3$ de triéthylamine puis concentré à sec sous pression réduite.

On obtient 15,7 g d'huile incolore. Celle-ci est chromatographiée sous pression, sur silice en éluant par un mélange chlorure de méthylène-acétonitrile (98-2).

On recueille 5,74 g d'un produit cristallisé F = 83 °C correspondant au diastéréoisomère R et 5,87 g d'une huile correspondant au diastéréoisomère S.

Le diastéréoisomère R présente les caractéristiques suivantes :

Spectre I.R. ($CHCl_3$)

Absence d'OH.

C = O γ-lactone 1795 $cm^{-1}$ max.

1748 $cm^{-1}$ épaul.

méthyle géminés 1385 $cm^{-1}$.

Spectre R.M.N. ($CDCl_3$)

Pic à 2,0 p.p.m. caractéristique des hydrogènes en 1 et 5 de la copule lactonique.

Pics à 1,15-1,18 p.p.m. caractéristiques des hydrogènes des méthyles de la copule lactonique.

Pic à 5,35 p.p.m. caractéristique de l'hydrogène en 4 de la copule lactonique.

Pic à 3,58 p.p.m. caractéristique de l'hydrogène en 1 du menthol.

Pics à 0,82-0,99 p.p.m. caractéristiques des hydrogènes du méthyle en 5 du menthol.

Pics à 0,75-0,99 p.p.m. caractéristiques des hydrogènes des méthyles de l'isopropyle.

Dichroïsme circulaire

Max. 224 nm $\Delta\varepsilon = -3{,}65$

$/\alpha/^{20}_D = -180° \pm 2{,}5°$ (C = 1,1 % benzène).

Le diastéréoisomère S présente les caractéristiques suivantes :

Spectre I.R. ($CHCl_3$)

Absence d'OH

C = O γ-lactone 1795 $cm^{-1}$

1748 $cm^{-1}$

méthyles géminés 1385 $cm^{-1}$.

Spectre R.M.N. ($CDCl_3$)

Pic à 2,0 p.p.m. caractéristique des hydrogènes en 1 et 5 de la copule lactonique.

Pics à 1,17-1,18 p.p.m. caractéristiques des hydrogènes des méthyles de la copule lactonique.

Pic à 5,18 p.p.m. caractéristique de l'hydrogène en 4 de la copule lactonique.

Pic à 3,43 p.p.m. caractéristique de l'hydrogène en 1 du menthol.

Pics à 0,75-0,98 p.p.m. caractéristiques des hydrogènes du méthyle en 5 du menthol.

Pics à 0,75-0,82 p.p.m. caractéristiques des hydrogènes des méthyles de l'isopropyle.

Dichroïsme circulaire
max. à 224-225 nm $\Delta\varepsilon = -3{,}20$
$/\alpha/^{20}_{D} = -53° \pm 2{,}5°$ (C = 0,42 % benzène).

Exemple 37 : (1R) (2S) (5R) 2 - isopropyl 5 - méthyl cyclohexanol

Pendant deux heures on chauffe au reflux sous agitation, 1 g de (1R, 5S) 6,6 - diméthyl (4R)/(1'R) (2'S) (5'R) 2' - isopropyl 5' - méthylcyclohexanoxy/ 3 - oxabicyclo /3.1.0/ hexan 2 - one, 10 $cm^3$ d'eau 10 $cm^3$ de dioxane et 100 mg d'acide paratoluène sulfonique. On concentre, dilue à l'eau, chasse le maximum de dioxane par distillation sous pression réduite. On extrait à l'éther isopropylique, sèche et concentre à sec sous pression réduite.

On obtient 430 mg d'huile. On chromatographie sur silice, élue par un mélange cyclohexane-acétate d'éthyle (7-3).

On isole 350 mg de produit identique au menthol naturel F < 50 °C.
$/\alpha/^{20}_{D} = -54{,}5° \pm 1°$ (C = 2 % éthanol).

Exemple 38 : (1S) (2R) (5S) 2 - isopropyl 5 - méthylcyclohexanol

Pendant 2 heures on chauffe au reflux sous agitation 5 g de (1R, 5S) 6,6 - diméthyl 4R-/(1'S) (2'R) (5'S) 2' - isopropyl 5' - méthylcyclohexanoxy/3 - oxabicyclo /3.1.0/ hexan 2 - one, 25 $cm^3$ de dioxane, 25 $cm^3$ d'eau et 100 mg d'acide paratoluène sulfonique. On distille sous pression réduite le maximum de dioxane, dilue à l'eau, extrait à l'éther isopropylique. La phase organique est séchée, concentrée à sec sous pression réduite pour donner 4,8 g d'huile. Après chromatographie sur silice on obtient 1,7 g de produit attendu F < 50 °C.
$/\alpha/^{20}_{D} = +46{,}5° \pm 2{,}5°$ (C = 0,35 % éthanol).

Schéma I

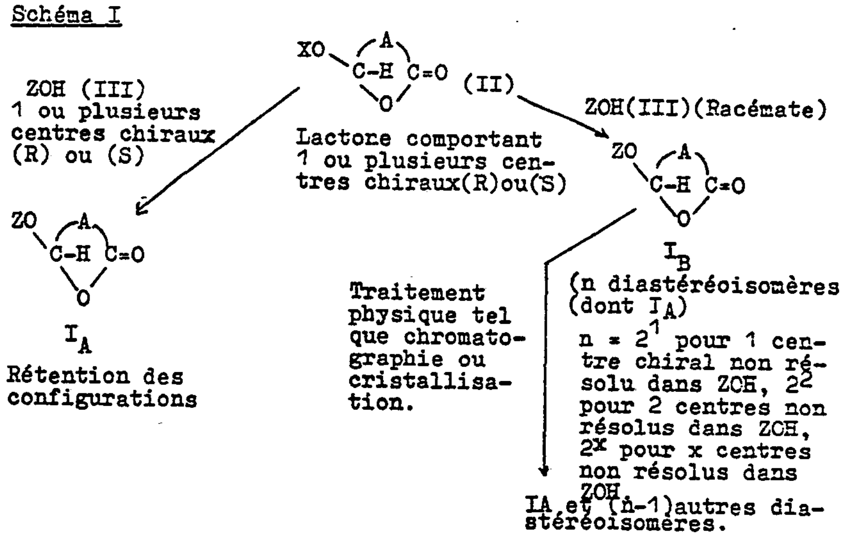


Schéma II

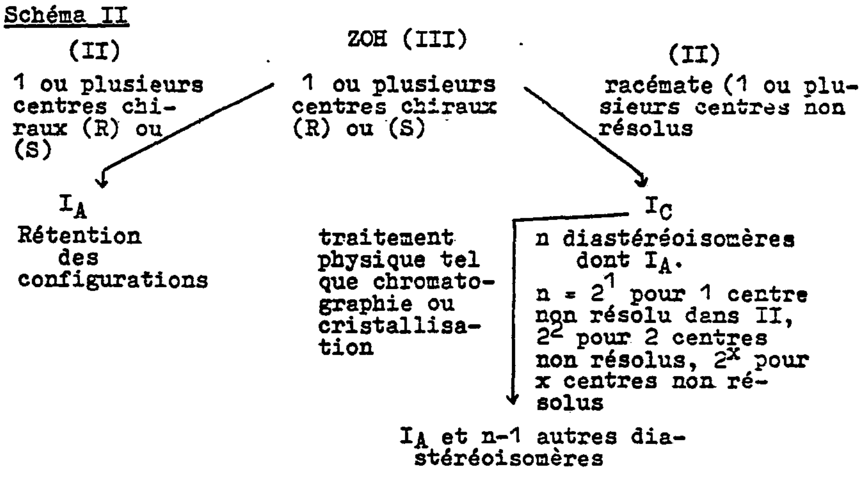

Schéma III

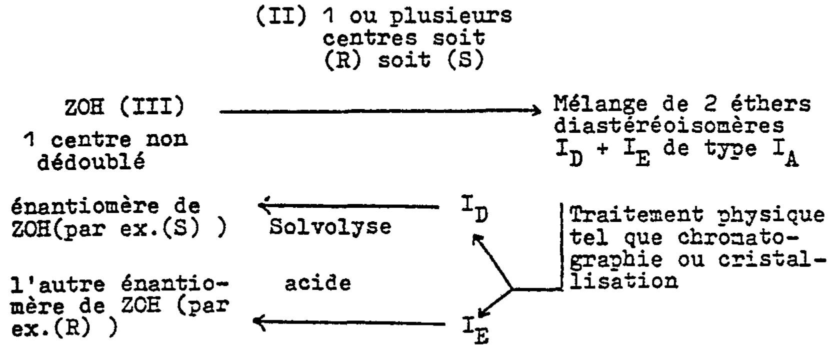


Schéma IV

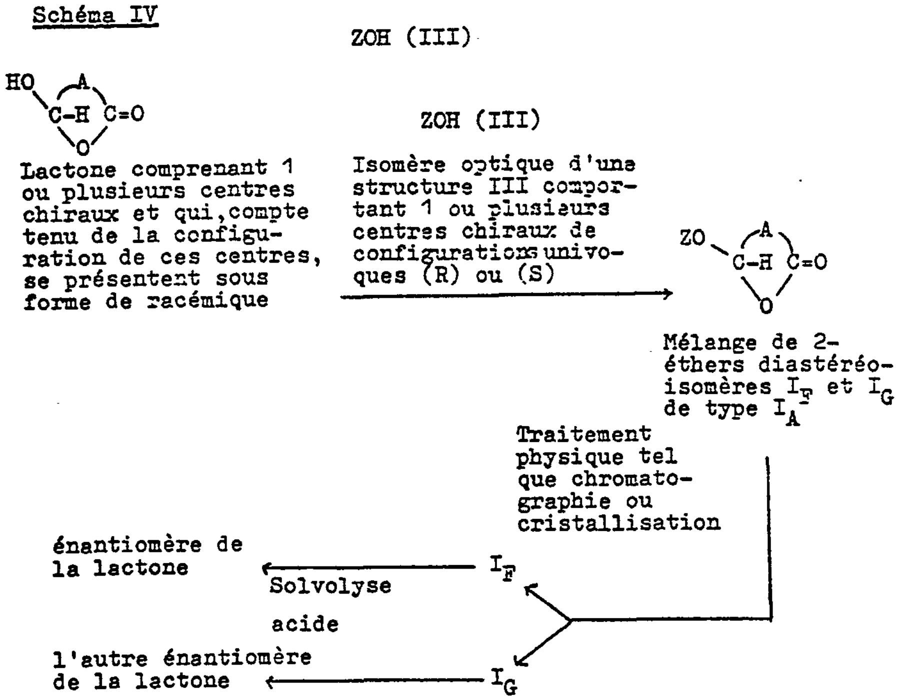


**Revendications**

1) Composés de formule générale I :

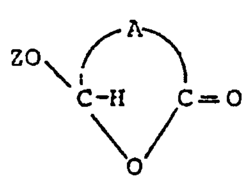

(I)

formule dans laquelle le symbole A représente une chaîne hydrocarbonée comportant de 1 à 10 chaînons, cette chaîne pouvant comporter un ou plusieurs hétéroatomes, une ou plusieurs insaturations, l'ensemble des chaînons constitutifs de la chaîne pouvant représenter un système mono ou polycyclique, y compris un système du type spiro ou endo, la chaîne A pouvant comporter un ou plusieurs atomes chiraux ou bien la copule lactonique pouvant présenter une chiralité due à la configuration spatiale dissymétrique de l'ensemble de la molécule et le symbole Z représente soit un reste d'alcool primaire, secondaire ou tertiaire, comportant au moins un carbone asymétrique, soit un reste phénolique substitué comportant au moins un carbone asymétrique, soit un reste alcoolique ou phénolique substitué dont la

chiralité est due à la configuration spatiale dissymétrique de l'ensemble de la molécule, étant entendu que Z ne peut représenter un radical (R) ou (S) α-cyano 3-phénoxy benzyle lorsque A représente une chaîne hydrocarbonée de structure

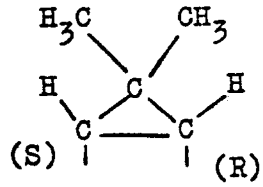

2) Composés selon la formule I de la revendication 1 dans laquelle la chaîne A comporte au moins un carbone asymétrique et dans laquelle les deux atomes ou radicaux différents qui substituent le ou les atomes de carbone asymétrique sont choisis indifféremment dans l'un ou l'autre des groupes suivants :

a) le groupe constitué par l'hydrogène, les atomes d'halogène, le groupement nitro, les radicaux alcoyles comportant de 1 à 10 atomes de carbone, les radicaux cycloalcoyles comportant de 3 à 6 atomes de carbone, le radical phényle, les radicaux phényles substitués par au moins un des éléments du groupe constitué par les atomes d'halogènes, les radicaux alcoyles comportant de 1 à 6 atomes de carbone, le groupement carboxyle, le groupement nitrile, le groupement -CHO, les groupements acyles, le groupement 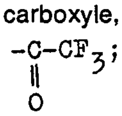 ; les groupements S-alc et O-alc dans lesquels alc représente un groupement alcoyle comportant de 1 à 6 atomes de carbone,

b) le groupe constitué par les radicaux :

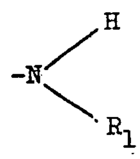

dans lesquels $R_1$ représente de l'hydrogène, un radical alcoyle comportant de 1 à 6 atomes de carbone,

le radical : 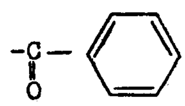 ,

le radical : 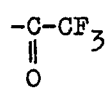 ,

le radical : 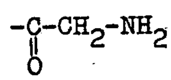 , le radical 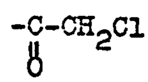 ,

ou un radical : 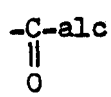 dans lequel alc représente un radical alcoyle comportant de 1 à 6 atomes de carbone,

c) le groupe constitué par les radicaux :

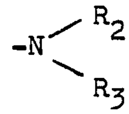

dans lequel $R_2$ et $R_3$, identiques ou différents, représentent un radical alcoyle comportant de 1 à 6 atomes de carbone, ou bien dans lequel $R_2$ et $R_3$ représentent ensemble avec l'atome d'azote auquel ils sont liés, un hétérocycle comportant 6 atomes, ou bien $R_2$ représente un groupement carboxyle et $R_3$ représente un radical benzyle.

3) Composés selon l'une des revendications 1 ou 2, caractérisés en ce que la chaîne A est une chaîne hydrocarbonée aliphatique comportant 2 ou 3 atomes de carbone.

4) Composés selon l'une des revendications 1 ou 2, caractérisés en ce que la chaîne A est une chaîne hydrocarbonée aliphatique interrompue par un hétéroatome.

5) Composés selon l'une des revendications 1 ou 2, caractérisés en ce que la chaîne A est une chaîne hydrocarbonée aliphatique comportant une double liaison.

6) Composés selon l'une des revendications 1 ou 2, caractérisés en ce que la chaîne A est une chaîne hydrocarbonée monocyclique comportant de 3 à 6 atomes de carbone, possédant, éventuellement, une insaturation.

7) Composés selon l'une des revendications 1 ou 2, caractérisés en ce que la chaîne A est une chaîne hydrocarbonée bicyclique comportant de 5 à 10 atomes de carbone, possédant, éventuellement, une insaturation.

8) Composés selon la revendication 2, caractérisés en ce que la chaîne A a pour structure :

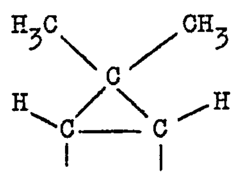

9) Composés selon la revendication 2, caractérisés en ce que la chaîne A a pour structure :

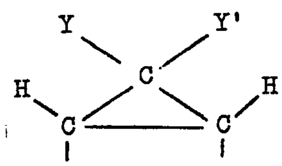

Y et Y', identiques ou différents, représentant des atomes d'hydrogène, de fluor, de chlore ou de brome ou un radical alcoyle comportant de 2 à 6 atomes de carbone ou bien Y et Y' pouvant former ensemble avec l'atome de carbone auquel ils sont liés un homocycle carboné comportant de 3 à 7 atomes de carbone.

10) Composés selon la revendication 2, caractérisés en ce que la chaîne A a pour structure :

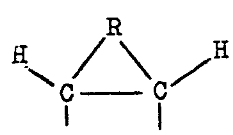

R représentant un atome d'oxygène, de soufre, un groupement -NH ou -NR', R' étant un radical alcoyle comportant de 1 à 6 atomes de carbone.

11) Composés selon l'une des revendications 1 ou 2, caractérisés en ce que le symbole Z a pour structure :

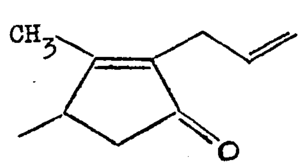

12) Composés selon l'une quelconque des revendications 1 ou 2, caractérisés en ce que le symbole Z représente le reste d'une cyanhydrine.

13) Composés selon l'une quelconque des revendications 1 ou 2, caractérisés en ce que le symbole Z a pour structure :

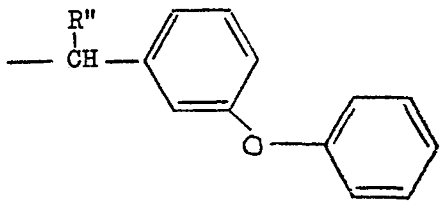

R'' représentant un radical alcoyle comportant de 1 à 6 atomes de carbone, un radical alcényle comportant de 2 à 6 atomes de carbone, un radical alcynyle comportant de 2 à 6 atomes de carbone ou un radical cyano.

14) Les composés ou mélanges de composés de formule générale I dont les noms suivent :

— mélange de (1R, 5S) 6,6 - diméthyl 4(R)-1/(S) - 2 - méthyl 4 - oxo 3 - (2 - propen - 1 - yl) cyclopent - 2 - enyloxy/3 - oxa bicyclo (3.1.0) hexan - 2 - one et de (1R, 5S) 6,6 - diméthyl 4(R)-/1(R) - 2 - méthyl 4 - oxo 3 - (2 - propen - 1 - yl) cyclopent - 2 - enyloxy/3 - oxa bicyclo (3.1.0) hexan - 2 - one,

— mélange de (1S, 5R) 6,6 - diméthyl 4(R)/1(S) - 2 - méthyl 4 - oxo 3 - (2 - propen - 1 - yl) cyclopent - 2 - enyloxy)/ 3 - oxa bicyclo (3.1.0) hexan - 2 - one et de (1S, 5R) 6,6 - diméthyl 4(R)/1(R) - 2 - méthyl 4 - oxo 3 - (2 - propen - 1 - yl) cyclopent - 2 - enyloxy/3 - oxa bicyclo (3.1.0) hexan - 2 - one,

— la (1R, 5S) 6,6 - diméthyl 4(R)/1(S) - 2 - méthyl 4 - oxo 3 - (2 - propen - 1 - yl) cyclopent - 2 -enyloxy/3 - oxa bicyclo (3.1.0) hexan - 2 -one,

— la (1S, 5R) 6,6 - diméthyl 4(R)/1 (R) - 2 - méthyl 4 - oxo 3 - (2 - propen - 1 - yl) cyclopent - 2 - enyloxy/3 - oxa bicyclo (3.1.0) hexan - 2 - one.

15) la (1R, 5S) 6,6 - diméthyl 4(R)/3',3' - diméthyl butyrolactone 2' - (S) oxy/3 - oxa bicyclo /3.1.0/ hexan - 2 - one,

— la (1R, 5S) 6,6 - diméthyl 4(R)/3',3' - diméthyl butyrolactone - 2'(R) oxy/3 - oxa bicyclo /3.1.0/ hexan - 2 - one, ainsi que le mélange de ces deux derniers composés,

16) la (3R) (3aR) (4S) (7R) (7aS) 3-/(1'R) (2'S) (5'R) 2 - isopropyl 5' - méthyl cyclohexanoxy/-tétrahydro 4,7 - méthano isobenzofuran - 1 - one,

— la (3R) (3aR) (4S) (7R) (7aS) 3-/(1'S) (2'R) (5'S) 2' - isopropyl 5' - méthyl cyclohexanoxy/ - tétrahydro 4,7 - méthano isobenzofuran - 1 - one, ainsi que le mélange de ces deux derniers composés,

— la (1R, 5S) 6,6 - diméthyl 4(R)-/(1'R) (2'S) (5'R) 2' - isopropyl 5' - méthyl cyclohexanoxy/3 - oxa bicyclo /3.1.0/ hexan - 2 - one,

— la (1R, 5S) 6,6 - diméthyl 4(R)-/(1'S) (2'R) (5'S) 2' - isopropyl 5' - méthyl cyclohexanoxy/3 - oxa bicyclo /3.1.0/ hexan - 2 - one, ainsi que le mélange de ces deux derniers composés.

17) Procédé de préparation des composés de formule gnérale I, tels que définis à la revendication 1, caractérisé en ce que l'on fait réagir un composé lactonique de formule générale II :

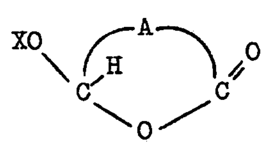

(II)

formule dans laquelle X représente un atome d'hydrogène ou un radical alcoyle comportant de 1 à 4 atomes de carbone et A conserve les significations de la revendication 1, en présence d'un acide, avec un alcool ou un phénol substitué de formule générale III :

ZOH (III)

formule dans laquelle Z conserve les significations de la revendication 1, pour obtenir

— ou bien un composé de formule (I), dénommé ($I_A$) dans lequel les atomes chiraux possèdent une configuration bien définie

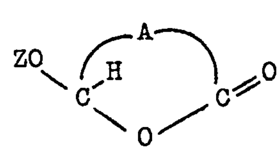

($I_A$)

lorsque la lactone et l'alcool ou le phénol possèdent un où plusieurs atomes chiraux de configurations bien définies,

— ou bien un mélange de diastéréoisomères, dénommé ($I_B$), lorsque la lactone est un isomère optique bien défini et les centres chiraux de l'alcool ou du phénol ne sont pas tous de configuration univoque,

— ou bien un mélange de diastéréoisomères, dénommé ($I_C$), lorsque l'alcool ou le phénol est un isomère optique bien défini et les atomes chiraux de la lactone ne sont pas tous de configuration univoque, puis sépare, par une méthode physique, les éthers diastéréoisomères contenus ou bien dans les mélanges du type ($I_B$), ou bien dans les mélanges du type ($I_C$) et, notamment, l'éther dénommé ($I_A$), dont les centres chiraux sont tous de configuration univoque.

18) Procédé de préparation des composés de formule générale I selon la revendication 17, caractérisé en ce que l'agent acide est choisi dans le groupe constitué par les acides sulfoniques, l'acide perchlorique et l'acide 5-sulfosalicylique.

19) Procédé de préparation des composés de formule générale I, selon l'une des revendications 17 ou 18, caractérisé en ce que l'eau formée lors de la condensation de l'alcool ou du phénol et du composé lactonique est éliminée par décantation azéotropique au reflux d'un solvant choisi dans le groupe constitué par les solvants chlorés, les hydrocarbures aromatiques ou aliphatiques et les éthers.

20) Procédé de préparation des composés de formule générale I selon l'une des revendications 17 ou 18, caractérisé en ce que la condensation de l'alcool ou du phénol et du composé lactonique est effectuée sous pression réduite et sans solvant.

21) Procédé de préparation des composés de formule générale I selon l'une des revendications 17 à 20, caractérisé en ce que la séparation des composés I diastéréoisomères est effectuée par cristallisation ou chromatographie.

22) Procédé de préparation selon l'une quelconque des revendications 17 à 21, caractérisé en ce que le composé de formule II est la lactone d'un acide cis 2,2 - diméthyl 3 - (dihydroxy - méthyl) cyclopropane - 1 - carboxylique racémique ou optiquement actif, le composé de formule III est la 1 - hydroxy 2 - méthyl 3 - (2 - propen - 1 - yl) cyclopent - 2 - ène 4 - one racémique ou optiquement active et en ce que la séparation éventuelle des composés I diastéréoisomères est effectuée par cristallisation dans un solvant organique.

23) Procédé de préparation selon l'une quelconque des revendications 17 à 21, caractérisé en ce que le composé de formule II est la lactone d'un acide cis 2,2 - diméthyl 3 - (dihydroxyméthyl) cyclopropane - 1 - carboxylique racémique ou optiquement actif, le composé de formule III est la 2 -hydroxy 3,3 - diméthyl butyrolactone (ou pantolactone) racémique ou optiquement active et la séparation éventuelle des composés diastéréoisomères est effectuée par chromatographie.

24) Procédé de préparation selon l'une quelconque des revendications 17 à 21, caractérisé en ce que le composé de formule II est la 3-hydroxy tétrahydro 4,7-méthano isobenzofuran-1-one, racémique ou optiquement active, le composé de formule III est le menthol racémique ou optiquement actif et la séparation éventuelle des composés diastéréoisomères est effectuée par chromatographie.

25) Procédé de préparation selon l'une quelconque des revendications 17 à 21, caractérisé en ce que le composé de formule II est la lactone d'un acide cis 2,2-diméth[illegible]-(dihydroxyméthyl) cyclopropane-1-carboxylique, racémique ou optiquement actif, le composé de formule III est le menthol racémique ou optiquement actif et la séparation éventuelle des composés diastéréoisomères est effectuée par chromatographie.

26) Application des composés de formule générale (I), tels que définis à la revendication 1, au dédoublement des composés de formule (II) ou (III), application qui consiste à faire réagir un composé lactonique de formule générale (II)

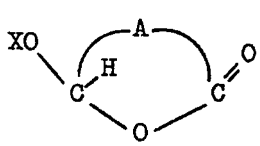

(II)

formule dans laquelle X représente un atome d'hydrogène ou un radical alcoyle comportant de 1 à 4 atomes de carbone et A conserve les significations de la revendication 1, en présence d'un acide, avec un alcool ou un phénol substitué de formule générale (III)

ZOH (III)

formule dans laquelle Z conserve les significations de la revendication 1, pour obtenir ou bien un mélange de diastéréoisomères, dénommés ($I_B$), lorsque la lactone est un isomère optique bien défini et les centres chiraux de l'alcool ou du phénol ne sont pas tous de configuration déterminée, ou bien un mélange de diastéréoisomères, dénommé ($I_C$), lorsque l'alcool ou le phénol est un isomère optique bien défini et les atomes chiraux de la lactone ne sont pas tous de configurations univoques, à séparer par une méthode physique les éthers diastéréoisomères contenus ou bien dans les mélangues du type ($I_B$) ou bien dans les mélanges du type ($I_C$) et, notamment, l'éther dénommé ($I_A$) dont les centres chiraux sont tous de

configuration univoque et caractérisée en ce que l'on soumet chacun des éthers diastéréoisomères ainsi séparés à une hydrolyse ou une alcoolyse en milieu acide, pour obtenir soit un composé du type II et les autres diastéréoisomères provenant éventuellement de l'existence de plusieurs centres asymétriques, soit un composé du type III et les autres diastéréoisomères provenant éventuellement de l'existence de plusieurs centres asymétriques, ces composés II et III et les divers diastéréoisomères correspondants, comportant des centres chiraux de configuration univoque, autrement dit, des centres chiraux dédoublés par rapport aux centres chiraux correspondants des alcools ou des phénols de départ, ou par rapport aux centres chiraux des composés lactoniques de départ.

27) Application selon la revendication 26 qui consiste à faire réagir la lactone de l'acide cis 2,2 - diméthyl 3S - (dihydroxyméthyl) cyclopropane - 1R - carboxylique, en présence d'un agent acide avec la 1(RS) - hydroxy 2 - méthyl 3 - (2 - propen - 1 - yl) cyclopent - 2 - èn 4 - one, à séparer par cristallisation dans l'isopropanol le (1R, 5S) 6,6 - diméthyl 4(R)/1(R) - 2 - méthyl 4 - oxo 3 - (2 - propen - 1 - yl) cyclopent - 2 - enyloxy/ 3 - oxa bicyclo (3.1.0) hexan - 2 - one du (1R, 5S) 6,6 - diméthyl 4(R)/1(S) - 2 - méthyl 4 - oxo 3 - (2 - propen - 1 - yl) cyclopent - 2 - enyloxy/3 - oxa bicyclo (3.1.0) hexan - 2 - one et est caractérisée en ce que l'on soumet ce dernier isomère « 1S » à une solvolyse en milieu acide pour obtenir la 1(S) - hydroxy 2 - méthyl 3 - (2 - propen - 1 - yl) cyclopent - 2 - èn - 4 - one.

28) Application selon la revendication 21, caractérisée en ce que la solvolyse est effectuée en milieu aqueux en présence d'acide chlorhydrique.

29) Application selon la revendication 27, caractérisée en ce que la solvolyse est effectuée dans le méthanol en présence d'acide paratoluène sulfonique.

30) Application selon la revendication 26, qui consiste à faire réagir la lactone de l'acide dl cis 2,2 - diméthyl 3S - (dihydroxyméthyl) cyclopropane - 1 - carboxylique, en présence d'un agent acide avec la 1(S) - hydroxy 2 - méthyl 3 - (2 - propen - 1 - yl) cyclopent - 2 - èn 4 - one, à séparer par cristallisation dans l'éther isopropylique le (1R, 5S) 6,6 - diméthyl 4(S)/1(S) - 2 - méthyl 4 - oxo 3 - (2 - propen - 1 - yl) cyclopent - 2 - enyloxy/3 - oxa bicyclo (3.1.0) hexan - 2 - one du (1R, 5S) 6,6 - diméthyl 4(R)/1(S) - 2 - méthyl 4 - oxo 3 - (2 - propen - 1 - yl) cyclopent - 2 - enyloxy/ 3 - oxa bicyclo (3.1.0) hexan - 2 - one, et est caractérisée en ce que l'on soumet ce dernier isomère « 4S » à une solvolyse en milieu acide pour obtenir la lactone de l'acide cis 2,2 - diméthyl 3S - (dihydroxyméthyl) cyclopropane - 1R - carboxylique.

31) Application selon la revendication 30, caractérisée en ce que l'agent acide utilisé est l'acide chlorhydrique.

32) Application selon la revendication 26, qui consiste à faire réagir la lactone de l'acide cis 2,2 - diméthyl 3S - (dihydroxyméthyl) cyclopropane - 1R - carboxylique, en présence d'un agent acide, avec la 2(R,S) hydroxy 3,3 - diméthylbutyrolactone, à séparer par chromatographie le (1R, 5S) 6,6 - diméthyl 4(R)/3',3' - diméthylbutyrolactone 2' - (S) - oxy/3 - oxa bicyclo /3.1.0/ hexan - 2 - one du (1R, 5S) 6,6 - diméthyl 4(R)-/3',3' - diméthylbutyrolactone 2'(R) oxy/3 - oxa bicyclo /3.1.0/ hexan - 2 - one, et est caractérisée en ce que l'on soumet l'un ou l'autre de ces isomères à une solvolyse, en milieu acide, pour obtenir la 2(S) - hydroxy 3,3 - diméthylbutyrolactone ou la 2(R) - hydroxy 3,3 - diméthylbutyrolactone selon l'isomère choisi.

33) Application selon la revendication 32, caractérisée en ce que la solvolyse est effectuée en milieu aqueux ou méthanolique, en présence d'acide paratoluène sulfonique.

34) Application selon la revendication 26, qui consiste à faire réagir la (3R) (3aR) (4S) (7R) (7aS) 3 - hydroxytétrahydro 4,7 - méthano isobenzofuran - 1 - one avec le (R,S) menthol, en présence d'un agent acide, à séparer par chromatographie la (3R) (3aR) (4S) (7R) (7aS) 3-/(1'R) (2'S) (5'R) 2' - isopropyl 5' - méthyl cyclohexanoxy/ tétrahydro 4,7 - méthano isobenzofuran - 1 - one de la (3R) (3aR) (4S) (7R) (7aS) 3-/(1'S) (2'R) (5'S) 2' - isopropyl 5' - méthylcyclohexanoxy/tétrahydro 4,7 - méthano isobenzofuran - 1 - one, et est caractérisée en ce que l'on soumet l'un ou l'autre de ces isomères à une solvolyse en milieu acide pour obtenir le (R) ou le (S) menthol.

35) Application selon la revendication 26, qui consiste à faire réagir la lactone de l'acide cis 2,2 - diméthyl 3S - (dihydroxyméthyl) cyclopropane - 1R - carboxylique, en présence d'un agent acide, avec le (R,S) menthol, à séparer par chromatographie la (1R, 5S) 6,6 - diméthyl (4R)/(1'R) (2'S) (5'R) 2' - isopropyl 5' - méthylcyclohexanoxy/3 - oxa bicyclo /3.1.0/ hexan - 2 - one de la (1R, 5S) 6,6 - diméthyl 4R-/(1'S) (2'R) (5'S) 2' - isopropyl 5' - méthyl cyclohexanoxy/3 - oxa bicyclo /3.1.0/ hexan - 2 - one, et est caractérisée en ce que l'on soumet l'un ou l'autre de ces isomères à une solvolyse en milieu acide pour obtenir le (R) ou le (S)-menthol.

36) Application selon la revendication 34 ou 35, caractérisée en ce que la solvolyse est effectuée en milieu aqueux, en présence d'acide paratoluène sulfonique.

**Claims**

1. Compounds of general formula I :

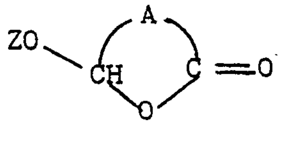

in which formula the symbol A represents a hydrocarbon chain containing from 1 to 10 links, this chain being capable of containing one or more heteroatoms, or one or more unsaturations, all of the links constituting the chain being capable of representing a mono or polycyclic system, including a system of the spiro or endo type, the chain A being capable of containing one or more chiral atoms or else the lactone component being capable of displaying a chirality due to the dissymmetric spacial configuration of the whole of the molecule and the symbol Z represents either a primary, secondary or tertiary alcohol containing at least one asymmetric carbon atom or a substituted phenolic residue containing at least one asymmetric carbon atom, or an alcoholic or substituted phenolic residue of which the chirality is due to the dissymmetric spacial configuration of the whole of the molecule, it being understood that Z cannot represent a (R) or (S) α-cyano 3-phenoxy benzyl radical when A represents a hydrocarbon chain of structure

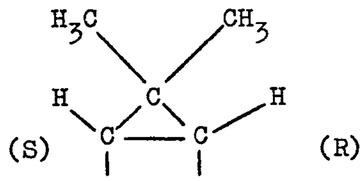

2. Compounds according to the formula I of Claim 1 in which the chain A contains at least one asymmetric carbon atom and in which the two different atoms or radicals which substitute the asymmetric carbon atom or atoms are selected indiscriminately from one or other of the following groups :

a) the group constituted by hydrogen, the halogen atoms, the nitro group, the alkyl radicals containing from 1 to 10 carbon atoms, the cycloalkyl radicals containing from 3 to 6 carbon atoms, the phenyl radical, the phenyl radicals substituted by at least one of the members of the group constituted by the halogen atoms, the alkyl radicals containing from 1 to 6 carbon atoms, the carboxyl group, the nitrile group, the group -CHO, the acyl groups, the group 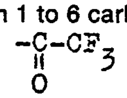 the groups S-alk and O-Alk in which alk represents an alkyl group containing from 1 to 6 carbon atoms,

b) the group constituted by the radicals :

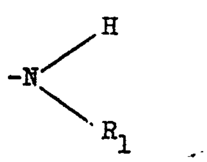

in which $R_1$ represents hydrogen, an alkyl radical containing from 1 to 6 carbon atoms,

the radical : 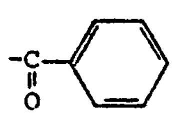

the radical : 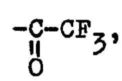

the radical : 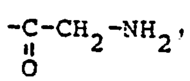 the radical : 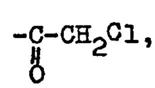

or a radical : 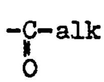 in which alk represents an alkyl radical containing from 1 to 6 carbon atoms,

c) the group constituted by the radicals :

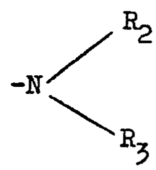

in which $R_2$ and $R_3$, being the same or different, represent an alkyl radical containing from 1 to 6 carbon atoms, or else in which $R_2$ and $R_3$ represent together with the nitrogen atom to which they are bonded, a heterocycle containing 6 atoms, or else $R_2$ represents a carboxyl group and $R_3$ represents a benzyl radical.

3. Compounds according to one of Claims 1 or 2, characterised in that the chain A is an aliphatic hydrocarbon chain containing 2 or 3 carbon atoms.

4. Compounds according to one of Claims 1 or 2, characterised in that the chain A is an aliphatic hydrocarbon chain interrupted by a heteroatom.

5. Compounds according to one of Claims 1 or 2, characterised in that the chain A is an aliphatic hydrocarbon chain containing one double bond.

6. Compounds according to one of Claims 1 or 2, characterised in that the chain A is a monocyclic hydrocarbon chain containing from 3 to 6 carbon atoms, possessing, possibly, one unsaturation.

7. Compounds according to one of Claims 1 or 2, characterised in that the chain A is a bicyclic hydrocarbon chain containing from 5 to 10 carbon atoms, possessing, possibly, one unsaturation.

8. Compounds according to Claim 2, characterised in that the chain A has as its structure :

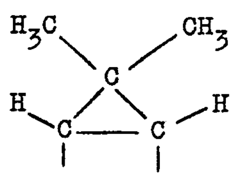

9. Compounds according to Claim 2, characterised in that the chain A has as its structure :

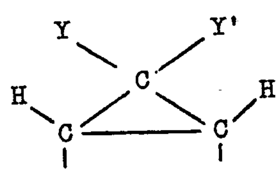

Y and Y', being the same or different, representing hydrogen, fluorine, chlorine or bromine atoms or an alkyl radical containing from 2 to 6 carbon atoms or else Y and Y' being capable of forming together with the carbon atom to which they are bonded a carbon homocycle containing from 3 to 7 carbon atoms.

10. Compounds according to Claim 2, characterised in that the chain A has as its structure :

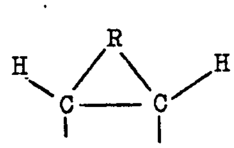

R representing an oxygen or sulphur atom, or a group -NH or -NR', R' being an alkyl radical containing from 1 to 6 carbon atoms.

11. Compounds according to one of Claims 1 or 2, characterised in that the symbol Z has as its structure :

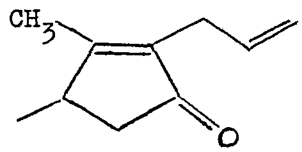

12. Compounds according to any one of Claims 1 or 2, characterised in that the symbol Z represents the residue of a cyanohydrin.

13. Compounds according to any one of Claims 1 or 2, characterised in that the symbol Z has as its structure :

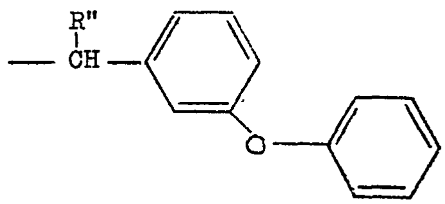

R" representing an alkyl radical containing from 1 to 6 carbon atoms, an alkenyl radical containing from 2 to 6 carbon atoms, an alkynyl radical containing from 2 to 6 carbon atoms or a cyano radical.

14. The compounds or mixtures of compounds of general formula I of which the names are as follows :

— mixture of (1R, 5S) 6,6 - dimethyl 4(R) - [1(S) - 2 - methyl 4 - oxo 3 - (2 - propen - 1 - yl) cyclopent - 2 - enyloxy] 3 - oxa bicyclo (3.1.0) hexan - 2 - one and (1R, 5S) 6,6 - dimethyl 4(R) - [1(R) - 2 - methyl 4 - oxo 3 - (2 - propen - 1 - yl) cyclopent - 2 - enyloxy] 3 - oxa - bicyclo (3.1.0) hexan - 2 - one,

— mixture of (1S, 5R) 6,6 - dimethyl 4(R) [1(S) - 2 - methyl 4 - oxo 3 - (2 - propen - 1 - yl) cyclopent - 2 - enyloxy] 3 - oxa bicyclo (3.1.0) hexan - 2 - one and (1S, 5R) 6,6 - dimethyl 4(R) [1(R) - 2 - methyl 4 - oxo 3 - (2 - propen - 1 - yl) cyclopent - 2 - enyloxy] 3 - oxa bicyclo (3.1.0) hexan - 2 - one,

— (1R, 5S) 6,6 - dimethyl 4(R) [1(S) - 2 - methyl 4 - oxo 3 - (2 - propen - 1 - yl) cyclopent - 2 - enyloxy] 3 - oxa bicyclo (3,1.0) hexan - 2 - one and

— (1S, 5R) 6,6 - dimethyl 4(R) [1(R) - 2 - methyl 4 - oxo 3 - (2 - propen - 1 - yl) cyclopent - 2 - enyloxy] 3 - oxa bicyclo (3.1.0) hexan - 2 - one.

15. (1R, 5S) 6,6 - dimethyl 4(R) [3',3' - dimethyl butyrolactone 2' - (S) oxy] 3 - oxa bicyclo [3.1.0] hexan - 2 - one,

— (1R, 5S) 6,6 - dimethyl 4(R) [3',3' - dimethyl butyrolactone 2'(R) oxyl 3 - oxa bicyclo [3.1.0] hexan - 2 - one, as well as the mixture of the two latter compounds.

16. (3) (3aR) (4S) (7R) (7aS) 3 - [(1'R) (2'S) (5'R) 2 - isopropyl 5' - methyl cyclohexanoxy] - tetrahydro 4,7 - methano isobenzofuran - 1 - one,

— (3R) (3aR) (4S) (7R) (7aS) 3 - [(1'S) (2'R) (5'S) 2' - isopropyl 5' - methyl cyclohexanoxy] - tetrahydro 4,7 - methano isobenzofuran - 1 - one, as well as the mixture of the two latter compounds,

— (1R, 5S) 6,6 - dimethyl 4(R) - [(1'R) (2'S) (5'R) 2' - isopropyl 5' - methyl cyclohexanoxy] 3 - oxa bicyclo [3.1.0] hexan 2 - one and

— (1R, 5S) 6,6 - dimethyl 4(R) - [(1'S) (2'R) (5'S) 2' - isopropyl 5' - methyl cyclohexanoxy] 3 -oxa bicyclo [3.1.0] hexan - 2 - one, as well as the mixture of the two latter compounds.

17. Process for preparing the compounds of general formula I, as defined in Claim 1, characterised in that a lactone compound of general formula II :

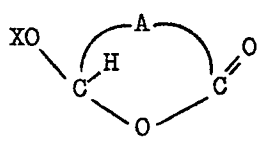

(II)

in which formula X represents a hydrogen atom or an alkyl radical containing from 1 to 4 carbon atoms and A keeps the meanings of Claim 1, is reacted, in the presence of an acid, with an alcohol or a substituted phenol of general formula III :

ZOH (III)

in which formula Z keeps the meanings of Claim 1, to obtain — either a compound of formula (I), called ($I_A$) in which the chiral atoms have a well — defined configuration :

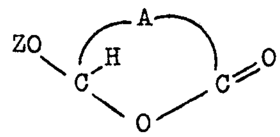

($I_A$)

when the lactone and the alcohol or the phenol have one or more chiral atoms of well-defined configurations,

— or a mixture of diastereoisomers, called ($I_B$), when the lactone is a well-defined optical isomer and the chiral centres of the alcohol or of the phenol are not all of unequivocal configuration,

— or a mixture of diastereoisomers, called ($I_C$), when the alcohol or the phenol is a well-defined optical isomer and the chiral atoms of the lactone are not all of unequivocal configuration,

then there are separated by a physical method the diastereoisomeric ethers contained either in the mixtures of the type ($I_B$) or the mixtures of the type ($I_C$) and, especially, the ether called ($I_A$), of which the chiral centres are all of unequivocal configuration.

18. Process for preparing the compounds of general formula I, according to Claim 17, characterised in that the acide agent is selected from the group constituted by the sulphonic acids, perchloric acid and 5-sulphosalicylic acid.

19. Process for preparing the compounds of general formula I, according to one of Claims 17 or 18, characterised in that the water formed during the condensation of the alcohol or of the phenol and of the lactone compound is removed by azeotropic decantation at reflux of a solvent selected from the group constituted by the chlorinated solvents, the aromatic or aliphatic hydrocarbons and the ethers.

20. Process for preparing the compounds of general formula I according to Claims 17 or 18, characterised in that the condensation of the alcohol or of the phenol and of the lactone compound is carried out under reduced pressure and without solvent.

21. Process for preparing the compounds of general formula I according to one of Claims 17 to 20, characterised in that the separation of the diastereoisomeric compounds I is carried out by crystallisation or chromatography.

22. Preparation process according to any one of Claims 17 to 21, characterised in that the compound of formula II is the racemic or optically-active lactone of a cis 2,2 - dimethyl 3 - (dihydroxymethyl) cyclopropane - 1 - carboxylic acid, the compound of formula III is racemic or optically-active 1 - hydroxy 2 - methyl 3 - (2 - propen - 1 - yl) cyclopent - 2 - en 4 - one and in that the possible separation of the diastereoisomeric compounds I is carried out by crystallisation from an organic solvent.

23. Preparation process according to any one of Claims 17 to 21, characterised in that the compound of formula II is the racemic or optically-active lactone of a cis 2,2 - dimethyl 3 - (dihydroxymethyl) cyclopropane - 1 - carboxylic acid, the compound of formula III is racemic or optically-active 2 - hydroxy 3,3 - dimethyl butyrolactone (or pantolactone) and the possible separation of the diastereoisomeric compounds is carried out by chromatography.

24. Preparation process according to any one of Claims 17 to 21, characterised in that the compound of formula II is racemic or optically-active 3 - hydroxy tetrahydro 4,7 - methano isobenzofuran - 1 - one, the compound of formula III is racemic or optically-active menthol and the possible separation of the diastereoisomeric compounds is carried out by chromatography.

25. Preparation process according to any one of Claims 17 to 21, characterised in that the compound of formula II is the racemic or optically-active lactone of a cis 2,2 - dimethyl 3 - (dihydroxymethyl) cyclopropane - 1 - carboxylic acid, the compound of formula III is racemic or optically-active menthol and the possible separation of the diastereoisomeric compounds is carried out by chromatography.

26. Use of compounds of general formula (I), as defined in Claim 1, in resolving the compounds of formula (II) or (III), which use consists in reacting a lactone compound of general formula (II) :

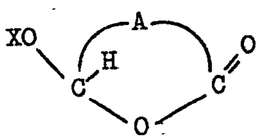

(II)

in which formula X represents a hydrogen atom or an alkyl radical containing from 1 to 4 carbon atoms

and A keeps the meanings of Claim 1, in the presence of an acid, with an alcohol or a substituted phenol of general formula (III) :

ZOH (III)

in which formula Z keeps the meanings of Claim 1, to obtain either a mixture of diastereoisomers, called ($I_B$), when the lactone is a well-defined optical isomer and the chiral centres of the alcohol or of the phenol are not all of fixed configuration, or a mixture of diastereoisomers, called ($I_C$), when the alcohol or the phenol is a well-defined optical isomer and the chiral atoms of the lactone are not all of unequivocal configurations, and consists in separating by a physical method the diastereoisomeric ethers contained either in the mixtures of the type ($I_B$) or in the mixtures of the type ($I_C$) and especially the ether called ($I_A$) of which the chiral centres are all of unequivocal configuration and is characterised in that each of the diastereoisomeric ethers thus separated is subjected to hydrolysis or alcoholysis in acidic medium, to obtain either a compound of the type II and the other diastereoisomers arising possibly from the existence of several asymmetric centres, or a compound of the type III and the other diastereoisomers arising possibly from the existence of several asymmetric centres, these compounds II and III and the various corresponding diastereoisomers containing chiral centres of unequivocal configuration, in other words chiral centres resolved with respect to the corresponding chiral centres of the starting alcohols or phenols, or with respect to the chiral centres of the starting lactone compounds.

27. Use according to Claim 26 which consists in reacting the lactone of cis 2,2 - dimethyl 3S - (dihydroxymethyl) cyclopropane - 1R - carboxylic acid, in the presence of an acid agent, with 1(RS) - hydroxy 2 - methyl 3 - (2 - propen - 1 - yl) cyclopent - 2 - en 4 - one and in separating by crystallisation from isopropanol (1R, 5S) 6,6 - dimethyl 4(R) - [1(R) - 2 - methyl 4 - oxo 3 - (2 - propen - 1 - yl) cyclopent - 2 - enyloxy] 3 - oxa bicyclo (3.1.0) hexan - 2 - one from (1R, 5S) 6,6 - dimethyl 4(R) [1(S) - 2 - methyl 4 - oxo 3 - (2 - propen - 1 - yl) cyclopent - 2 - enyloxy] 3 - oxa bicyclo (3.1.0) hexan - 2 - one and is characterised in that the latter isomer « 1S » is subjected to solvolysis in acidic medium to obtain 1(S) - hydroxy 2 - methyl 3 - (2 - propen - 1 - yl) cyclopent - 2 - en - 4 - one.

28. Use according to Claim 21, characterised in that the solvolysis is carried out in aqueous medium in the presence of hydrochloric acid.

29. Use according to Claim 27, characterised in that the solvolysis is carried out in methanol in the presence of paratoluene sulphonic acid.

30. Use according to Claim 26, which consists in reacting the lactone of dl cis 2,2 - dimethyl 3S - (dihydroxymethyl) cyclopropane - 1 - carboxylic acid, in the presence of an acid agent, with 1(S) - hydroxy - 2 - methyl 3 - (2 - propen - 1 - yl) cyclopent - 2 - en 4 - one and in separating by crystallisation from isopropyl ether (1R, 5S) 6,6 - dimethyl 4(S) [1(S) - 2 - methyl 4 - oxo 3 - (2 - propen - 1 - yl) cyclopent - 2 - enyloxy] 3 - oxa bicyclo (3.1.0) hexan - 2 - one from (1R, 5S) 6,6 - dimethyl 4(R) [1(S) - 2 - methyl 4 - oxo 3 - (2 - propen - 1 - yl) cyclopent - 2 - enyloxy] 3 - oxa bicyclo (3.1.0) hexan - 2 - one, and is characterised in that the latter isomer « 4S » is subjected to solvolysis in acidic medium to obtain the lactone of cis 2,2 - dimethyl 3S - (dihydroxy-methyl) cyclopropane - 1R - carboxylic acid.

31. Use according to Claim 30, characterised in that the acid agent used is hydrochloric acid.

32. Use according to Claim 26 which consists in reacting the lactone of cis 2,2 - dimethyl 3S - (dihydroxymethyl) cyclopropane - 1R - carboxylic acid, in the presence of an acid agent, with 2(R,S) hydroxy 3,3 - dimethylbutyrolactone and in separating by chromatography (1R, 5S) 6,6 - dimethyl 4(R) [3',3' - dimethylbutyrolactone 2' - (S) - oxy] 3 - oxa - bicyclo [3.1.0] hexan - 2 - one from (1R, 5S) 6,6 - dimethyl - 4(R) - [3',3' - dimethylbutyrolactone 2'(R) oxy] 3 - oxa bicyclo [3.1.0] hexan - 2 - one and is characterised in that one or other of these isomers is subjected to solvolysis, in acidic medium, to obtain 2(S) - hydroxy 3,3 - dimethylbutyrolactone or 2(R) - hydroxy 3,3 - dimethylbutyrolactone according to the isomer selected.

33. Use according to Claim 32, characterised in that the solvolysis is carried out in aqueous or methanolic medium, in the presence of paratoluene sulphonic acid.

34. Use according to Claim 26, which consists in reacting (3R) (3aR) (4S) (7R) (7aS) 3 - hydroxytetrahydro 4,7 - methano isobenzofuran - 1 - one with (R, S) menthol, in the presence of an acid agent and in separating by chromatography (3R) (3aR) (4S) (7R) (7aS) 3 - [(1'R) (2'S) (5'R) 2' - isopropyl 5' - methyl cyclohexanoxy] tetrahydro 4,7 - methano isobenzofuran - 1 - one from (3R) (3aR) (4S) (7R) (7aS) 3 - [(1'S) (2'R) (5'S) 2' - isopropyl 5' - methylcyclohexanoxy] tetrahydro 4,7 - methano isobenzofuran - 1 - one and is characterised in that one or other of these isomers is subjected to solvolysis in acidic medium to obtain (R) or (S) menthol.

35. Use according to Claim 26, which consists in reacting the lactone of cis 2,2 - dimethyl 3S - (dihydroxymethyl) cyclopropane - 1R - carboxylic acid, in the presence of an acid agent, with (R, S) menthol and in separating by chromatography (1R, 5S) 6,6 - dimethyl (4R) [(1'R) (2'S) (5'R) 2' - isopropyl 5' - methylcyclohexanoxy] 3 - oxa bicyclo [3.1.0] hexan - 2 - one from (1R, 5S) 6,6 - dimethyl 4R - [(1'S) (2'R) (5'S) 2' - isopropyl 5' - methyl cyclohexanoxy] 3 - oxa bicyclo [3.1.0] hexan - 2 - one and is characterised in that one or other of these isomers is subjected to solvolysis in acidic medium to obtain (R) or (S) - menthol.

36. Use according to Claim 34 or 35, characterised in that the solvolysis is carried out in aqueous medium, in the presence of paratoluene sulphonic acid.

**Ansprüche**

1. Verbindungen der allgemeinen Formel I :

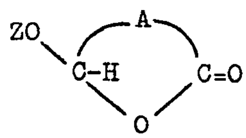

(I)

worin A eine Kohlenwasserstoffkette mit 1 bis 10 Kettengliedern bedeutet, wobei diese Kette ein oder mehrere Heteroatome und eine oder mehrere Unsättigungen enthalten kann und wobei die Gesamtheit der die Kette bildenden Kettenglieder ein mono- oder polycyclisches System einschließlich eines Systems von spiro- oder endo-Typ darstellen kann, wobei die Kette A ein oder mehrere chirale Atome oder auch eine lactonische Verknüpfung enthalten kann, die auf Grund der asymmetrischen räumlichen Konfiguration eine Chiralität aufweisen kann, und das Symbol Z antweder einen Rest eines primären, sekundären oder tertiären Alkohols mit zumindest einem asymmetrischen Kohlenstoffatom oder einen substituierten phenolischen Rest mit zumindest einem asymmetrischen Kohlenstoffatom oder einen alkoholischen oder substituierten phenolischen Rest bedeutet, dessen Chiralität auf die asymmetrische räumliche Konfiguration der Gesamtheit des Moleküls zurüchkzuführen ist, vorausgesetzt, daß Z keinen (R) - oder (S) - α - Cyano - 3 - phenoxybenzylrest bedeutet, wenn A eine Kohlenwasserstoffkette mit der Struktur

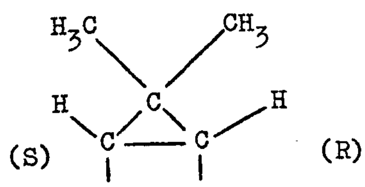

bedeutet.

2. Verbindungen gemäß Anspruch 1, worin die Kette A zumindest ein asymmetrisches Kohlenstoffatom enthält und worin die beiden verschiedenen Atome oder Reste, die das oder die asymmetrischen Kohlenstoffatome substituieren in gleicher Weise ausgewählt sind unter einer oder mehreren der folgenden Gruppen :

a) der Gruppe bestehend aus Wasserstoff, den Halogenatomen, der Nitrogruppe, den Alkylresten mit 1 bis 10 Kohlenstoffatomen, den Cycloalkylresten mit 3 bis 6 Kohlenstoffatomen, dem Phenylrest, den Phenylresten, substituiert durch zumindest eines der Elemente der Gruppe bestehend aus den Halogenatomen, den Alkylresten mit 1 bis 6 Kohlenstoffatomen, der Carboxylgruppe, der Nitrilgruppe, der Gruppe -CHO, den Acylgruppen, der Gruppe 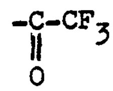 den Gruppen S-Alk und O-Alk, worin die Reste Alk eine Alkylgruppe mit 1 bis 6 Kohlenstoffatomen bedeuten,

b) der Gruppe bestehend aus den Resten :

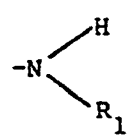

worin $R_1$ ein Wasserstoffatom oder einen Alkylrest mit 1 bis 6 Kohlenstoffatomen bedeutet,

dem Rest : 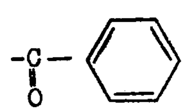

dem Rest : 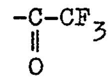

dem Rest : 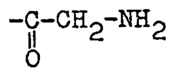 , dem Rest : 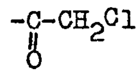 oder

einem Rest : 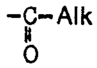 , worin Alk einen Alkylrest mit 1 bis 6 Kohlenstoffatomen darstellt,

c) der Gruppe bestehend aus den Resten :

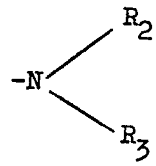

worin $R_2$ und $R_3$, die gleich oder verschieden sein können, einen Alkylrest mit 1 bis 6 Kohlenstoffatomen bedeuten oder aber $R_2$ und $R_3$ gemeinsam mit dem Stickstoffatom, an das sie gebunden sind, einen Heterocyclus mit 6 Atomen darstellen oder aber $R_2$ eine Carboxylgruppe bedeutet und $R_3$ einen Benzylrest darstellt.

3. Verbindungen gemäß einem der Ansprüche 1 oder 2, dadurch gekennzeichnet, daß die Kette A eine aliphatische Kohlenwasserstoffkette mit 2 oder 3 Kohlenstoffatomen ist.

4. Verbindungen gemäß einem der Ansprüche 1 oder 2, dadurch gekennzeichnet, daß die Kette A eine durch ein Heteroatom unterbrochene aliphatische Kohlenwasserstoffkette ist.

5. Verbindungen gemäß einem der Ansprüche 1 oder 2, dadurch gekennzeichnet, daß die Kette A eine aliphatische Kohlenwasserstoffkette mit einer Doppelbindung ist.

6. Verbindungen gemäß einem der Ansprüche 1 oder 2, dadurch gekennzeichnet, daß die Kette A eine monocyclische Kohlenwasserstoffkette mit 3 bis 6 Kohlenstoffatomen ist, die gegebenenfalls eine Unsättigung enthält.

7. Verbindungen gemäß einem der Ansprüche 1 oder 2, dadurch gekennzeichnet, daß die Kette A eine bicyclische Kohlenwasserstoffkette mit 5 bis 10 Kohlenstoffatomen ist, die gegebenenfalls eine Unsättigung enthält.

8. Verbindungen gemäß Anspruch 2, dadurch gekennzeichnet, daß die Kette A die folgende Struktur besitzt :

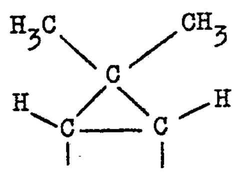

9. Verbindungen gemäß Anspruch 2, dadurch gekennzeichnet, daß die Kette A die folgende Struktur besitzt :

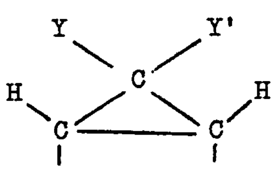

worin Y und Y', die gleich oder verschieden sein können, Wasserstoff, Fluor-, Chlor- oder Bromatome oder einen Alkylrest mit 2 bis 6 Kohlenstoffatomen darstellen oder auch Y und Y' gemeinsam mit dem Kohlenstoffatom, an das sie gebunden sind, einen Kohlenstoffhomocyclus bilden können, der 3 bis 7 Kohlenstoffatome enthält.

10. Verbindungen gemäß Anspruch 2, dadurch gekennzeichnet, daß die Kette A die folgende Struktur besitzt :

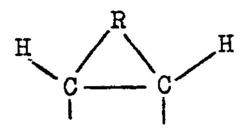

worin R ein Sauerstoffatom, ein Schwefelatom, eine Gruppe -NH oder -NR' darstellt, worin R' einen Alkylrest mit 1 bis 6 Kohlenstoffatomen bedeutet.

11. Verbindungen gemäß einem der Ansprüche 1 oder 2, dadurch gekennzeichnet, daß das Symbol Z die folgende Struktur besitzt :

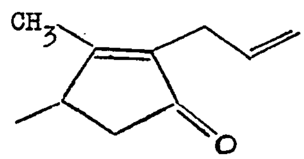

12. Verbindungen gemäß einem der Ansprüche 1 oder 2, dadurch gekennzeichnet, daß das Symbol Z den Rest eines Cyanhydrins darstellt.

13. Verbindungen gemäß einem der Ansprüche 1 oder 2, dadurch gekennzeichnet, daß das Symbol Z die folgende Struktur besitzt :

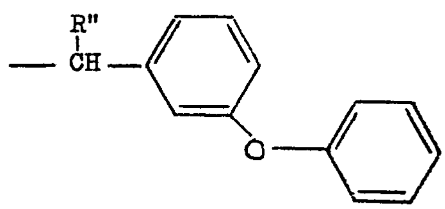

worin R" einen Alkylrest mit 1 bis 6 Kohlenstoffatomen, einen Alkenylrest mit 2 bis 6 Kohlenstoffatomen, einen Alkinylrest mit 2 bis 6 Kohlenstoffatomen oder einen Cyanorest bedeutet.

14. Verbindungen oder Mischungen von Verbindungen der allgemeinen Formel I mit den folgenden Bezeichnungen :

Mischung von (1R, 5S) - 6,6 - Dimethyl - 4(R) - [1(S) - 2 - methyl - 4 - oxo - 3 - (2 - propen - 1 - yl) - cyclopent - 2 - enyloxy] - 3 - oxa - bicyclo [3.1.0] hexan - 2 - on und (1R, 5S) - 6,6 - Dimethyl - 4(R) - [1(R) - 2 - methyl - 4 - oxo - 3 (2 - propen - 1 - yl) - cyclopent - 2 - enyloxy] - 3 - oxa - bicyclo [3.1.0] hexan - 2 - on,

Mischung von (1S, 5R) - 6,6 - Dimethyl - 4(R) - [1(S) - 2 - methyl - 4 - oxo - 3 - (2 - propen - 1 - yl) - cyclopent - 2 - enyloxy] - 3 - oxa - bicyclo [3.1.0] hexan - 2 - on und (1S, 5R) - 6,6 - Dimethyl - 4(R) - [1(R) - 2 - methyl - 4 - oxo - 3 - (2 - propen - 1 - yl) - cyclopent - 2 - enyloxy] - 3 - oxa - bicyclo [3.1.0] hexan - 2 - on,

(1R, 5S) - 6,6 - Dimethyl - 4(R) - [1(S) - 2 - methyl - 4 - oxo - 3 - (2 - propen - 1 - yl) - cyclopent - 2 - enyloxy] - 3 - oxa - bicyclo [3.1.0] hexan - 2 - on,

(1S, 5R) - 6,6 - Dimethyl - 4(R) - [1(R) - 2 - methyl - 4 - oxo - 3 - (2 - propen - 1 - yl) - cyclopent - 2 - enyloxy] - 3 - oxa - bicyclo [3.1.0] hexan - 2 - on.

15. (1R, 5S) - 6,6 - Dimethyl - 4(R) - [3',3' - dimethylbutyrolacton - 2'(S) - oxy] - 3 - oxabicyclo [3.1.0] hexan - 2 - on,

(1R, 5S) - 6,6 - Dimethyl - 4(R) - [3',3' - dimethylbutyrolacton - 2'(R) - oxy] - 3 - oxabicyclo [3.1.0] hexan - 2 - on,

sowie die Mischung dieser beiden letztgenannten Verbindungen.

16. (3R) (3aR) (4S) (7R) (7aS) - 3 - [(1'R) (2'S) (5'R) - 2 - Isopropyl - 5' - methylcyclohexanoxy] - tetrahydro - 4,7 - methano - isobenzofuran - 1 - on,

(3R) (3aR) (4S) (7R) (7aS) - 3 - [(1'S) (2'R) (5'S) - 2' - Isopropyl - 5' - methylcyclohexanoxy] - tetrahydro - 4,7 - methano - isobenzofuran - 1 - on,

sowie die Mischung dieser beiden letztgenannten Verbindungen,

(1R, 5S) - 6,6 - Dimethyl - 4(R) - [(1'R) (2'S) (5'R) - 2' - isopropyl - 5' - methylcyclohexanoxy] - 3 - oxabicyclo [3.1.0] hexan - 2 - on.

(1R, 5S) - 6,6 - Dimethyl - 4(R) - [(1'S) (2'R) (5'S) - 2' - isopropyl - 5' - methylcyclohexanoxy] - 3 - oxabicyclo [3.1.0] hexan - 2 - on,
sowie die Mischung dieser beiden letztgenannten Verbindungen.

17. Verfahren zur Herstellung von Verbindungen der allgemeinen Formel I gemäß Anspruch 1, dadurch gekennzeichnet, daß man eine Lactonverbindung der allgemeinen Formel II :

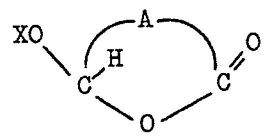

(II)

worin X ein Wasserstoffatom oder einen Alkylrest mit 1 bis 4 Kohlenstoffatomen bedeutet und A die im Anspruch 1 angegebene Bedeutung besitzt, in Gegenwart einer Säure mit einem Alkohol oder einem substituierten Phenol der allgemeinen Formel III :

ZOH (III)

worin Z die in Anspruch 1 angegebene Bedeutung besitzt, umsetzt, um entweder eine Verbindung der Formel I, als Verbindung der Formel $I_A$ bezeichnet, zu erhalten, worin die chiralen Atome eine gut definierte Konfiguration besitzen :

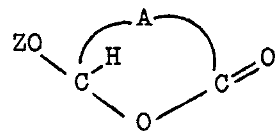

($I_A$)

wenn das Lacton und der Alkohol oder das Phenol ein oder mehrere chirale Atome mit gut definierten Konfigurationen besitzen,

oder ein Gemisch von Diastereomeren, bezeichnet als $I_B$, zu erhalten, wenn das Lacton ein gut definiertes optisches Isomeres ist und die chiralen Zentren des Alkohols oder Phenols nicht sämtlich eine eindeutige Konfiguration aufweisen,

oder eine Mischung von Diastereomeren, als $I_C$ bezeichnet, zu erhalten, wenn der Alkohol oder das Phenol ein gut definiertes optisches Isomeres ist und die chiralen Atome des Lactons nicht sämtlich eine eindeutige Konfiguration besitzen,

und anschließend nach einer physikalischen Methode die entweder in den Mischungen von Typ $I_B$ oder in den Mischungen von Typ $I_C$ enthaltenen diastereomeren Äther und insbesondere den als $I_A$ bezeichneten Äther, deren Chiralitätszentren sämtlich eine eindeutige Konfiguration aufweisen, trennt.

18. Verfahren zur Herstellung von Verbindungen der allgemeinen Formel I gemäß Anspruch 17, dadurch gekennzeichnet, daß das saure Mittel unter den Sulfonsäuren, der Perchlorsäure und der 5-Sulfosalicylsäure ausgewählt wird.

19. Verfahren zur Herstellung von Verbindungen der allgemeinen Formel I gemäß einem der Ansprüche 17 oder 18, dadurch gekennzeichnet, daß das während der Kondensation des Alkohols oder des Phenols und der Lactonverbindung gebildete Wasser durch azeotrope Dekantation unter Rückfluß eines Lösungsmittels, ausgewählt unter den chlorierten Lösungsmitteln, den aromatischen oder aliphatischen Kohlenwasserstoffen und den Äthern, entfernt wird.

20. Verfahren zur Herstellung von Verbindungen der allgemeinen Formel I gemäß Anspruch 17 oder 18, dadurch gekennzeichnet, daß die Kondensation des Alkohols oder Phenols und der Lactonverbindung unter vermindertem Druck ohne Lösungsmittel durchgeführt wird.

21. Verfahren zur Herstellung von Verbindungen der allgemeinen Formel I gemäß einem der Ansprüche 17 bis 20, dadurch gekennzeichnet, daß die Trennung der diastereomeren Verbindungen der Formel I durch Kristallisation oder Chromatographie bewirkt wird.

22. Verfahren gemäß einem der Ansprüche 17 bis 21, dadurch gekennzeichnet, daß die Verbindung der Formel II das Lacton einer racemischen oder optisch aktiven cis - 2,2 - Dimethyl - 3 - (dihydroxymethyl) - cyclopropan - 1 - carbonsäure ist, die Verbindung der Formel III das racemische oder optisch aktive 1 - Hydroxy - 2 - methyl - 3 - (2 - propen - 1 - yl) - cyclopent - 2 - en - 4 - on ist und daß die etwaige Trennung der diastereomeren Verbindungen I durch Kristallisation in einem organischen Lösungsmittel durchgeführt wird.

23. Verfahren gemäß einem der Ansprüche 17 bis 21, dadurch gekennzeichnet, daß die Verbindung der Formel II das Lacton einer racemischen oder optisch aktiven cis - 2,2 - Dimethyl - 3 - (dihydroxymethyl) - cyclopropan - 1 - carbonsäure ist, die Verbindung der Formel III das racemische oder optsich aktive 2 - Hydroxy - 3,3 - dimethyl - butyrolacton (oder Pantolacton) ist und daß die etwaige Trennung der diastereomeren Verbindungen durch Chromatographie erfolgt.

24. Verfahren gemäß einem der Ansprüche 17 bis 21, dadurch gekennzeichnet, daß die Verbindung

der Formel II das racemische oder optisch aktive 3 - Hydroxytetrahydro - 4,7 - methanoisobenzofuran - 1 - on ist, die Verbindung der Formel III racemisches oder optisch aktives Menthol ist und die etwaige Trennung der diastereomeren Verbindungen durch Chromatographie erfolgt.

25. Verfahren gemäß einem der Ansprüche 17 bis 21, dadurch gekennzeichnet, daß die Verbindung der Formel II das racemische oder optisch aktive Lacton einer cis - 2,2 - Dimethyl - 3 - (dihydroxymethyl) - cyclopropan - 1 -carbonsäure ist, die Verbindung der Formel III racemisches oder optisch aktives Menthol ist und die etwaige Trennung der diastereomeren Verbindugen durch Chromatographie erfolgt.

26. Verwendung der Verbindungen der allgemeinen Formel I gemäß Anspruch 1 zur Spaltung von Verbindungen der Formel II oder III, die darin besteht, daß man eine Lactonverbindung der allgemeinen Formel II

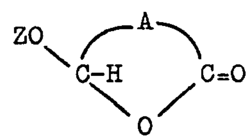

(II)

worin X ein Wasserstoffatom oder einen Alkylrest mit 1 bis 4 Kohlenstoffatomen bedeutet und A die in Anspruch 1 angegebenen Bedeutungen besitzt, in Gegenwart einer Säure mit einem Alkohol oder einem substituierten Phenol der allgemeinen Formel III

ZOH (III)

worin Z die in Anspruch 1 angegebene Bedeutung besitzt, umsetzt, um
entweder eine Mischung von Diastereomeren, als $I_B$ bezeichnet, zu erhalten, wenn das Lacton ein gut definiertes optisches Isomeres ist und die chiralen Zentren des Alkohols oder des Phenols nicht sämtlich eine bestimmte Konfiguration aufweisen,

oder eine Mischung von Diastereomeren, bezeichnet als $I_C$ zu erhalten, wenn der Alkohol oder das Phenol ein gut definiertes optisches Isomeres ist und die chiralen Atome des Lactons nicht sämtlich eindeutige Konfiguration besitzen,

daß man nach einer physikalischen Methode die entweder in den Mischungen von Typ $I_B$ oder in den Mischungen vom Typ $I_C$ enthaltenen diastereomeren Äther und insbesondere den als $I_A$ bezeichneten Äther, deren chirale Zentren sämtlich eine eindeutige Konfiguration aufweisen, trennt und die dadurch gekennzeichnet ist, daß man einen jeden der so getrennten diastereomeren Äther einer Hydrolyse oder einer Alkoholyse in saurem Milieu unterzieht, um entweder eine Verbindung vom Typ II und die weiteren gegebenenfalls dem Vorliegen mehrerer Asymmetriezentren entstammenden Diastereomeren oder eine Verbindung vom Typ III und die weiteren gegebenenfalls dem Vorliegen mehrerer Asymmetriezentren entstammenden Diastereomeren zu erhalten, wobei diese Verbindungen II und III und die entsprechenden verschiedenen Diastereomeren chirale Zentren mit eindeutiger Konfiguration bzw. gespaltene chirale Zentren in bezug auf die entsprechenden chiralen Zentren der Ausgangs-Alkohole oder -Phenole oder in bezug auf die chiralen Zentren der Ausgangs-Lactonverbindungen enthalten.

27. Verwendung gemäß Anspruch 26, bei der man das Lacton der cis - 2,2 - Dimethyl - 3S - (dihyroxymethyl) - cyclopropan - 1R - carbonsäure in Gegenwart eines sauren Mittels mit dem 1(RS) - Hydroxy - 2 - methyl - 3 - (2 - propen - 1 - yl) - cyclopent - 2 - en - 4 - on umsetzt, durch Kristallisation in Isopropanol das (1R, 5S) - 6,6 - Dimethyl - 4(R) - [1(R) - 2 - methyl - 4 - oxo - 3 - (2 - propen - 1 - yl) - cyclopent - 2 - enyloxy] - 3 - oxabicyclo [3.1.0] hexan - 2 - on vom (1R, 5S) - 6,6 - Dimethyl - 4(R) - [1(S) - 2 - methyl - 4 - oxo - 3 - (2 - propen - 1 - yl) - cyclopent - 2 - enyloxy] - 3 - oxabicyclo [3.1.0] hexan - 2 - on trennt und die dadurch gekennzeichnet ist, daß man dieses letztgenannte « 1S » - Isomere einer Sovolyse in saurem Milieu unterzieht, um das 1S - Hydroxy - 2 - methyl - 3 - (2 - propen - 1 - yl) - cyclopent - 2 - en - 4 - on zu erhalten.

28. Verwendung gemäß Anspruch 21, dadurch gekennzeichnet, daß die Solvolyse in wäßrigem Milieu in Gegenwart von Chlorwasserstoffsäure durchgeführt wird.

29. Verwendung gemäß Anspruch 27, dadurch gekennzeichnet, daß man die Solvolyse in Methanol in Gegenwart von p-Toluolsulfonsäure durchführt.

30. Verwendung gemäß Anspruch 26, bei der man das Lacton der dl - cis - 2,2 - Dimethyl - 3S - (dihydroxymethyl) - cyclopropan - 1 - carbonsäure in Gegenwart eines sauren Mittels mit dem 1(S) - Hydroxy - 2 - methyl - 3 - (2 - propen - 1 - yl) - cyclopent - 2 - en - 4 - on umsetzt, durch Kristallisation in Isopropyläther das (1R, 5S) - 6,6 - Dimethyl - 4(S) - [1(S) - 2 - methyl -'4 - oxo - 3 - (2 - propen - 1 - yl) cyclopent - 2 - enyloxy] - 3 - oxabicyclo [3.1.0] hexan - 2 - on vom (1R, 5S) - 6,6 - Dimethyl - 4 - (R) - [1(S) - 2 - methyl - 4 - oxo - 3 (2 - propen - 1 - yl) - cyclopent - 2 - enyloxy] - 3 - oxabicyclo [3.1.0] hexan - 2 - on abtrennt und die dadurch gekennzeichnet ist, daß man dieses letztgenannte « 4S » - Isomere einer Solvolyse in saurem Milieu unterzieht, um das Lacton der cis - 2,2 - Dimethyl - 3S - (dihydroxymethyl) - cyclopropan - 1R - carbonsäure zu erhalten.

31. Verwendung gemäß Anspruch 30, dadurch gekennzeichnet, daß das verwendete saure Mittel die Chlorwasserstoffsäure ist.

32. Verwendung gemäß Anspruch 26, bei der man das Lacton der cis - 2,2 - Dimethyl - 3S - (dihydroxymethyl) - cyclopropan - 1R - carbonsäure in Gegenwart eines sauren Mittels mit dem 2(R,S) - Hydroxy - 3,3 - dimethylbutyrolacton umsetzt, durch Chromatographie das (1R, 5S) - 6,6 - Dimethyl - 4(R) - [3',3' - dimethylbutyrolacton - 2' - (S) - oxy] - 3 - oxabicyclo - 3.1.0] hexan - 2 - on vom (1R, 5S) - 6,6 - Dimethyl - 4(R) - [3',3' - dimethylbutyrolacton - 2' - (R) - oxy] - 3 - oxabicyclo [3.1.0] hexan - 2 - on trennt und die dadurch gekennzeichnet ist, daß man das eine oder andere dieser Isomeren in saurem Milieu einer Solvolyse unterzieht, um das 2(S) - Hydroxy - 3,3 - dimethylbutyrolacton oder das 2(R) - Hydroxy - 3,3 - dimethylbutyrolacton gemäß dem ausgewählten Isomeren zu erhalten.

33. Verwendung gemäß Anspruch 32, dadurch gekennzeichnet, daß die Solvolyse in wäßrigen oder methanolischem Milieu in Gegenwart von p - Toluolsulfonsäure durchgeführt wird.

34. Verwendung gemäß Anspruch 26, bei der man das (3R) (3aR) (4S) (7R) (7aS) - 3 - Hydroxytetrahydro - 4,7 - methanoisobenzofuran - 1 - on mit dem (R,S) - Menthol in Gegenwart eines sauren Mittels umsetzt, durch Chromatographie das (3R) (3aR) (4S) (7R) (7aS) - 3 - [(1'R) (2'S) (5'R) - 2' - Isopropyl - 5' - methylcyclohexanoxy] - tetrahydro - 4,7 - methano - isobenzofuran - 1 - on vom (3R) (3aR) (4S) (7R) (7aS) - 3 - [(1'S) (2'R) (5'S) - 2' - Isopropyl - 5' - methylcylohexanoxy] - tetrahydro - 4,7 - methanoisobenzofuran - 1 - on trennt und die dadurch gekennzeichnet ist, daß man das eine oder andere dieser Isomeren in saurem Milieu einer Solvolyse unterzieht, um das (R) - oder (S) - Menthol zu erhalten.

35. Verwendung gemäß Anspruch 26, bei der man das Lacton der cis - 2,2 - Dimethyl - 3S (dihydroxymethyl) - cyclopropan - 1R - carbonsäure in Gegenwart eines sauren Mittels mit dem (R,S) - Menthol umsetzt, durch Chromatographie das (1R, 5S) - 6,6 - Dimethyl - (4R) - [(1'R) (2'S) (5'R) - 2' - isopropyl - 5' - methylcyclohexanoxy] - 3 - oxabicyclo [3.1.0] hexan - 2 - on vom (1R, 5S) - 6,6 - Dimethyl - 4R - [(1'S) (2'R) (5'S) - 2' - isopropyl - 5' - methylcyclohexan - oxy] - 3 - oxabicyclo [3.1.0] hexan - 2 - on trennt und die dadurch gekennzeichnet ist, daß man das eine oder andere dieser Isomeren einer Solvolyse in saurem Milieu unterzieht, um das (R) - oder (S) - Menthol zu erhalten.

36. Verwendung gemäß Anspruch 34 oder 35, dadurch gekennzeichnet, daß die Solvolyse in wäßrigen Milieu in Gegenwart von p-Toluolsulfonsäure durchgeführt wird.